# EUROPÄISCHE PATENTSCHRIFT

(11) **EP 3 662 483 B1**
(45) Veröffentlichungstag und Bekanntmachung des Hinweises auf die Patenterteilung: **20.04.2022**
(21) Anmeldenummer: 17749184.2
(22) Anmeldetag: 04.08.2017
(51) Int. Cl.: G16H 10/60

(54) **PATIENTENREKRUTIERUNGSSYSTEM**
PATIENT RECRUITMENT SYSTEM
SYSTÈME DE RECRUTEMENT DES PATIENTS

(43) Veröffentlichungstag der Anmeldung: 10.06.2020
(73) Patentinhaber: Clinerion Ltd., 4051 Basel (CH)
(72) Erfinder: WALTER, Andreas, 76532 Baden-Baden (DE); ARONSKY, Dominik, 8803 Rüschlikon (CH); BODENMANN, Bernhard, 4102 Binningen (CH); STRZEPKA, Lukasz, 5603 Staufen (CH); CLAESSON, Ulf, 8907 Wettswil (CH)
(74) Vertreter: Daub, Thomas
(86) Internationale Anmeldenummer: PCT/EP2017/069859
(87) Internationale Veröffentlichungsnummer: WO 2019/025015

(56) Entgegenhaltungen:
- WO-A1-2009/065043
- WO-A1-2017/042396
- WO-A1-2017/042396
- WO-A2-2010/149948
- WO-A2-2010/149948
- US-A1- 2003 039 362
- US-A1- 2003 039 362
- US-A1- 2011 253 139
- US-A1- 2011 253 139
- US-A1- 2014 316 793
- US-A1- 2014 316 793

## Beschreibung

### Stand der Technik

Die Erfindung betrifft ein Patientenrekrutierungssystem nach dem Anspruch 1 und ein Verfahren zur Rekrutierung von Patienten nach dem Anspruch 6.

Es ist aus der US 2014/316793 A1 bereits ein Patientenrekrutierungssystem, insbesondere für klinische Studien, mit zumindest einer Datenbankumgebung, welches zumindest ein Datenbankmodul aufweist, das zumindest dazu vorgesehen ist, zumindest Patientendatensätze mit zumindest einem Behandlungsverlauf zumindest eines Patienten in einer Patientendatenbank abzuspeichern, mit zumindest einem Patientenrekrutierungsserver, welcher zumindest ein Patientenrekrutierungsmodul aufweist, welches dazu vorgesehen ist, Datensätze, insbesondere Behandlungsverläufe, zu durchsuchen und zumindest mit zumindest einem Rekrutierungsmerkmal abzugleichen, und mit einer Datentransporteinheit zu einem Datentransfer, insbesondere zumindest eines Behandlungsverlaufs, zwischen der Datenbankumgebung und dem Patientenrekrutierungsserver, vorgeschlagen worden. Zudem sind aus der WO 2017/042396 A1, der WO 2010/149948 A2 und der WO 2009/065043 A1 weitere Datenverwaltungssysteme, die im Zusammenhang mit klinischen Studien verwendet werden, bekannt. In der US 2011/253139 A1 und in der US 200/3039362 A1 sind zudem weitere medizinische Datenverarbeitungssysteme beschrieben.

Die Aufgabe der Erfindung besteht insbesondere darin, ein gattungsgemäßes System mit verbesserten Eigenschaften hinsichtlich einer Datenübertragung, insbesondere in Bezug auf einen Datenschutz, bereitzustellen. Die Aufgabe wird erfindungsgemäß durch die Merkmale der Patentansprüche 1 und 6 gelöst, während vorteilhafte Ausgestaltungen und Weiterbildungen der Erfindung den Unteransprüchen entnommen werden können.

### Vorteile der Erfindung

Die Erfindung geht aus von einem Patientenrekrutierungssystem, insbesondere für klinische Studien, mit zumindest einer Datenbankumgebung, welche zumindest ein Datenbankmodul aufweist, das zumindest dazu vorgesehen ist, zumindest Patientendatensätze mit zumindest einem Behandlungsverlauf zumindest eines Patienten in einer Patientendatenbank abzuspeichern, mit zumindest einem Patientenrekrutierungsserver, welcher zumindest ein Patientenrekrutierungsmodul aufweist, welches dazu vorgesehen ist, Datensätze, insbesondere Behandlungsverläufe, zu durchsuchen und zumindest mit zumindest einem Rekrutierungsmerkmal abzugleichen, und mit einer Datentransporteinheit zu einem Datentransfer, insbesondere zumindest eines Behandlungsverlaufs, zwischen der Datenbankumgebung, insbesondere einem Patientenrekrutierungsmodul der Datenbankumgebung, und dem Patientenrekrutierungsserver, insbesondere einem Patientenrekrutierungsmodul des Patientenrekrutierungsservers.

Es wird vorgeschlagen, dass die Datentransporteinheit ein Beschränkungsmodul aufweist, welches den Datentransfer auf von der Datenbankumgebung ausgehende Outbound Verbindungen beschränkt. Dadurch kann vorteilhaft eine Datenübertragung, insbesondere hinsichtlich einer Datensicherheit, verbessert werden. Vorteilhaft können dadurch sensible Daten, insbesondere Patientendaten, sicher übertragen werden. Insbesondere ist die Datentransporteinheit auf der Seite der Datenbankumgebung frei von Inbound Ports und weist ausschließlich Outbound Ports auf, wodurch vorteilhaft sichergestellt werden kann, dass Verbindungen zwischen der Datenbankumgebung und dem Patientenrekrutierungsserver immer von der Seite der Datenbankumgebung initiiert werden. Dadurch kann vorteilhaft erreicht werden, dass der durch die Datentransporteinheit übermittelte Datentransfer für die Seite der Datenbankumgebung vollständig transparent ist. Zudem kann vorteilhaft ein unautorisierter Zugriff auf die Datenbankumgebung mittels der Datentransporteinheit, beispielsweise durch "hacking", unterbunden werden. Vorteilhaft kann mittels des Patientenrekrutierungssystems eine anonymisierte Identifizierung von passenden Probanden für eine klinische Studie erleichtert werden, wodurch vorteilhaft innerbetriebliche Prozesse in einem Spital optimiert und/oder beschleunigt werden können. Dadurch können vorteilhaft Kosten gesenkt werden.

Unter einer "Datenbankumgebung" soll insbesondere ein, insbesondere kontrollierendes, Computersystem verstanden werden. Vorzugsweise umfasst die Datenbankumgebung zumindest einen Teil eines Spitalinformationssystems, insbesondere mit elektronisch abgespeicherten Patientenakten und/oder elektronisch abgespeicherten Krankheitsverläufen, und/oder zumindest ein zu dem Spitalinformationssystem zugehöriges Datenbankmodul mit zumindest einer Patientendatensätze und/oder Patienteninformationen enthaltenden Patientendatenbank. Vorzugsweise ist die Datenbankumgebung als ein dediziertes Computersystem oder als zumindest ein Teil eines dedizierten Computersystems ausgebildet, könnte aber alternativ auch zumindest teilweise als virtueller Server, als mehrere verteilt angeordnete Server oder als ein Server in einer, insbesondere privaten und/oder öffentlichen, Cloud ausgebildet sein. Erfindungsgemäß ist die Datenbankumgebung innerhalb eines speziell gesicherten und/oder zugangsbeschränkten Bereichs des Intranets des Spitalinformationssystems angeordnet. Vorteilhaft befindet sich die Datenbankumgebung in einem, vorzugsweise zugangsbeschränkten und/oder besonders gesicherten Raum eines Spitals. Außerdem umfasst die Datenbankumgebung vorzugsweise weitere Komponenten, insbesondere zu einer Anonymisierung von Patientendatensätzen mittels einem Anonymisierungsmodul, zu einer Initiierung eines Datentransports mittels einer Datentransporteinheit, zu einer Initiierung zumindest einer Anfrage von Datensätzen von einem Server und/oder einem Computersystem außerhalb der Datenbankumgebung mittels eines Patientenrekrutierungsmoduls der Datenbankumgebung und/oder zu einer Berechnung von zu Datensätzen zugehörigen Identifikatoren mittels eines Identifikatormoduls. Vorzugsweise ist der Zugang zu der Datenbankumgebung und/oder zu zumindest einer, bevorzugt allen Komponente/n der Datenbankumgebung geschützt, insbesondere passwortgeschützt. Vorzugsweise ist die Datenbankumgebung mittels zumindest einer Firewall und/oder bevorzugt mittels weiterer Schutzkomponenten, wie beispielsweise einem "Intrusion Detection System" (IDS) und/oder einem "Intrusion Prevention System" (IPS) geschützt. In der Patientendatenbank sind insbesondere Patientendatensätze unverschlüsselt, oder vorzugsweise verschlüsselt, abspeicherbar. Ein Patientendatensatz umfasst insbesondere zumindest einen Patientendatensatz, wie beispielsweise zumindest eine durchgeführte Behandlung, z.B. eine Operation, zumindest eine Diagnose, z.B. eine Krankheitsdiagnose, eine symptomatische Diagnose und/oder eine bestimmte physiologische Größe wie Blutdruck, Lungenvolumen, Körperfettanteil und/oder dergleichen, zumindest einen Medikationsverlauf, z.B. eine Medikamentendosis, ein Medikationszeitraum, eine Medikamentenunverträglichkeit und/oder dergleichen, zumindest eine Prozedur, zumindest Labordaten, wie beispielsweise Blutwerte, und/oder zumindest personenspezifische Daten wie beispielsweise Alter, Gewicht, Größe, Geschlecht, Herkunft, Geburtsdatum, Name, Informationen zur Krankenversicherung und/oder Adresse. Vorzugsweise entspricht ein Patientendatensatz zumindest teilweise einer elektronischen Patientenakte eines Spitals. Vorzugsweise ist jedem Patientendatensatz ein eindeutiger Patientenidentifikator zugeordnet. Unter einem "Datensatz" soll insbesondere ein Patientendatensatz oder ein anonymisierter Patientendatensatz verstanden werden. Unter einem "Behandlungsverlauf" soll insbesondere eine, insbesondere zeitliche, Abfolge von zumindest einer Behandlung, zumindest einer Diagnose und/oder zumindest einer Medikation verstanden werden. Insbesondere umfasst der Patientendatensatz eine Menge aller bekannten Behandlungsverläufe eines Patienten. Vorzugsweise umfasst der anonymisierte Patientendatensatz eine anonymisierte Menge aller Behandlungsverläufe eines Patienten.

Unter einem "Patientenrekrutierungsserver" soll insbesondere ein Server verstanden werden, der zur Patientenrekrutierung anhand von, insbesondere anonymisierten Patientendatensätzen zumindest einer Gesundheitsinstitution, beispielsweise einer ambulatorischen Klinik, einer Arztpraxis und/oder vorzugsweise eines Spitals, vorgesehen ist. Vorzugsweise kommuniziert ein Patientenrekrutierungsmodul der Datenbankumgebung mit einem Patientenrekrutierungsmodul des Patientenrekrutierungsservers mittels einer, ausschließlich von der Datenbankumgebung ausgehende Outbound Verbindungen zulassenden, dem Fachmann als sinnvoll erscheinenden Datentransporteinheit, vorzugsweise einer Server-to-Server Kommunikation. Vorzugsweise sind der Patientenrekrutierungsserver und die Datenbankumgebung verteilt, insbesondere an verschiedenen Orten, angeordnet. Vorzugsweise ist der Patientenrekrutierungsserver als ein dedizierter Server ausgebildet, könnte aber alternativ auch als virtueller Server, als mehrere verteilt angeordnete Server oder als ein Server in einer Cloud ausgebildet sein. Vorzugsweise ist der Patientenrekrutierungsserver innerhalb des Intranets des Spitalinformationssystems angeordnet. Vorzugsweise ist zwischen dem Patientenrekrutierungsserver und der Datenbankumgebung zumindest eine Firewall zwischengeschaltet. Vorzugsweise befindet sich der Patientenrekrutierungsserver hinter einer Firewall des Spitalinformationssystems. Erfindungsgemäß sind die Datenbankumgebung, insbesondere Netze der Datenbankumgebung, und der Patientenrekrutierungsserver, insbesondere Netze des Patientenrekrutierungsservers, logistisch voneinander getrennt ausgebildet. Eine Kommunikation zwischen der Datenbankumgebung und dem Patientenrekrutierungsserver ist vorzugsweise verschlüsselt, beispielsweise mittels einem SSL und/oder einem HTTPS Protokoll. Insbesondere befindet sich der Patientenrekrutierungsserver in einer sogenannten demilitarisierten Zone eines Spitalinformationssystems. Vorzugsweise ist der Patientenrekrutierungsserver dazu vorgesehen, insbesondere anonymisierte, Patientendatensätze, anhand von Rekrutierungsmerkmalen zumindest einem, dem Fachmann als sinnvoll erscheinenden Vorgang zuzuordnen, vorteilhaft klinischen Studien und/oder Vorhersagen über Krankheitsverläufe. Das Patientenrekrutierungsmodul der Datenbankumgebung kann vorteilhaft als ein separater Server innerhalb der Datenbankumgebung ausgebildet sein. Alternativ kann das Anonymisierungsmodul vorteilhaft in einem Server der Datenbankumgebung, beispielsweise einen Datenbankserver der Datenbankumgebung, integriert sein. Unter einem "Rekrutierungsmerkmal" soll insbesondere eine Vorgabe verstanden werden, die von einer Teilmenge der Patientendatensätze erfüllt wird. Vorzugsweise können mittels des Rekrutierungsmerkmals Patientendatensätze in die Teilmenge aufgenommen oder aus der Teilmenge ausgeschlossen werden. Vorzugsweise ist das Rekrutierungsmerkmal als eine Vorgabe ausgebildet, welche zumindest ein Kriterium für eine Teilnahme an zumindest einer klinischen Studie beinhaltet, beispielsweise zumindest eine bestimmte physiologische Eigenschaft, Diagnose und/oder Medikation. Das Rekrutierungsmerkmal kann insbesondere ein Höchstalter, ein Mindestalter, zumindest eine Diagnose, zumindest einen Laborwert, zumindest einen Sensorwert, zumindest eine Medikamentendosis, zumindest einen Medikationsverlauf, zumindest Vitaldaten, wie beispielsweise ein Blutdruck, und/oder ein Gewicht umfassen. Vorzugsweise umfasst ein Rekrutierungsmerkmal eine Kombination von einer Mehrzahl an Kriterien.

Unter einer "Datentransporteinheit" soll insbesondere eine Einheit verstanden werden, welche dazu vorgesehen ist Informationen, insbesondere Patientendatensätze, vorzugsweise anonymisierte Patientendatensätze von einem Sender der Datentransporteinheit zu einem Empfänger der Datentransporteinheit zu übertragen. Insbesondere können Sender und Empfänger Teile voneinander verschiedener Computersysteme und/oder Server ausbilden, wobei vorzugsweise Sender und Empfänger innerhalb eines gemeinsamen Intranets angeordnet sind. Eine Datenübertragung zwischen Sender und Empfänger erfolgt vorzugsweise innerhalb eines Festkörpers, beispielsweise mittels einer, insbesondere seriellen oder parallelen, Kabelverbindung und/oder drahtlos. Insbesondere kann die Datentransporteinheit ein Beschränkungsmodul aufweisen, welches dazu vorgesehen ist einen Datentransfer zu überwachen und/oder anhand bestimmter Kriterien, wie beispielsweise einer Datenflussrichtung, einer Datenmenge, einer Datenbeschaffenheit und/oder einer Datenart einzuschränken. Das Beschränkungsmodul ist insbesondere als zumindest eine Firewall und/oder als eine weitere einen Datenverkehr beeinflussende Programmierung der Datentransporteinheit ausgebildet. Unter einer "Outbound Verbindung" soll insbesondere eine Verbindung der Datentransporteinheit, insbesondere zwischen zumindest einem Teil der Datenbankumgebung und dem Patientenrekrutierungsserver, verstanden werden, welche ausschließlich von einer Datenbankumgebungsseite initiierbar ist. Erfindungsgemäß ist ein Datentransport zwischen zumindest einem Teil der Datenbankumgebung und dem Patientenrekrutierungsserver mittels einer Outbound Verbindung ausschließlich von der Datenbankumgebungsseite steuerbar. Insbesondere ist die Datentransporteinheit frei von offenen Ports auf der Datenbankumgebungsseite. Insbesondere kann der Patientenrekrutierungsserver ausschließlich Daten an die Datenbankumgebung senden, welche von der Datenbankumgebungsseite zuvor angefordert wurden. Vorzugsweise ist der Datentransfer mittels der Outbound Verbindung, insbesondere zwischen der Datenbankumgebung und dem Patientenrekrutierungsserver, frei von unanonymisierten Datensätzen, insbesondere unanonymisierten Patientendatensätzen. Vorzugsweise ist der Datentransfer mittels der Outbound Verbindung, insbesondere zwischen der Datenbankumgebung und dem Patientenrekrutierungsserver, frei von Identifikatoren und/oder Merkmalen, aus welchen, insbesondere unter Einhaltung von Datenschutzverordnungen und -gesetzen, eine Person und/oder ein Patient eindeutig identifizierbar ist. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Ferner weist die Datenbankumgebung erfindungsgemäß ein Anonymisierungsmodul auf, welches dazu vorgesehen ist, insbesondere vor einem Datentransfer mittels der Datentransporteinheit zu dem Patientenrekrutierungsserver, Patientendatensätze in anonymisierte Patientendatensätze, insbesondere anonymisierte Behandlungsverläufe, umzuwandeln. Vorteilhaft kann ein umfangreicher Datenschutz ermöglicht werden, wodurch insbesondere eine hohe Sicherheit erzielt werden kann. Dadurch kann vorteilhaft eine Bereitschaft zur Anwendung und/oder Akzeptanz des Patientenrekrutierungssystems, insbesondere durch Spitäler, Patienten und/oder Ärzte erhöht werden, wodurch insbesondere eine Organisation von klinischen Studien verbessert, erleichtert und/oder beschleunigt werden kann. Mittels einer Anonymisierung kann vorteilhaft eine Datenübertragung, insbesondere hinsichtlich einer Datensicherheit, verbessert werden. Vorteilhaft können dadurch sensible Daten, insbesondere Patientendaten, sicher übertragen werden. Das Anonymisierungsmodul ist insbesondere als Teil der Datenbankumgebung ausgebildet. Das Anonymisierungsmodul ist insbesondere dazu vorgesehen Erkennungsmerkmale aus Patientendatensätzen zu entfernen, welche zu einer, insbesondere eindeutigen, Identifizierung eines Patienten führen können. Erkennungsmerkmale umfassen beispielsweise Name, Geburtsdatum, Geburtsort, Adresse, Informationen über Familienmitglieder, Sozialversicherungsnummer und/oder andere eindeutig einer Person zuordenbare Identifikationsmerkmale, wie beispielweise biometrische Informationen, eindeutige Identifikatoren der Patientendatensätze und/oder Identifikatoren der Behandlungsverläufe innerhalb der Patientendatenbank. Ein anonymisierter Patientendatensatz ist insbesondere frei von Erkennungsmerkmalen. Es ist vorstellbar, dass das Anonymisierungsmodul einem Patientendatensatz einen anonymisierten statischen und/oder zeitlich veränderlichen Identifikator zuordnet, vorzugsweise ist ein Patientendatensatz jedoch nach einer Bearbeitung durch das Anonymisierungsmodul frei von Identifikatoren. Insbesondere ein zeitlich veränderlicher Identifikator kann beispielsweise bei jeder Bearbeitung und/oder Aktualisierung eines, insbesondere anonymisierten Patientendatensatzes, neu zugeordnet und/oder berechnet werden. Das Anonymisierungsmodul kann vorteilhaft als ein separater Server innerhalb der Datenbankumgebung ausgebildet sein. Alternativ kann das Anonymisierungsmodul vorteilhaft in einem Server der Datenbankumgebung, beispielsweise einen Datenbankserver der Datenbankumgebung, integriert sein. Vorzugsweise ist jeder Patientendatenbank ein Anonymisierungsmodul zugeordnet. Vorzugsweise ist das Anonymisierungsmodul zumindest zwischen dem Datenbankmodul und dem Beschränkungsmodul zwischengeschaltet. Vorteilhaft weist das Datenbankmodul eine, insbesondere von der Patientendatenbank getrennte, anonymisierte Patientendatenbank auf, in welcher anonymisierte Patientendaten abgespeichert sind und/oder abspeicherbar sind.

Zudem sind die durch das Anonymisierungsmodul anonymisierten Patientendatensätze, insbesondere unter Berücksichtigung von Datensicherheitsvorschriften und/oder eines Arztgeheimnisses, erfindungsgemäß frei von eindeutig einem Patienten zuordenbaren Identifikatoren und/oder Erkennungsmerkmalen. Dadurch kann vorteilhaft eine besonders vollständige Anonymisierung erreicht werden, wodurch vorteilhaft ein Datenschutz verbessert werden kann. Dadurch kann vorteilhaft eine Bereitschaft zur Anwendung und/oder Akzeptanz des Patientenrekrutierungssystems, insbesondere durch Spitäler, Patienten und/oder Ärzte erhöht werden, wodurch insbesondere eine Organisation von klinischen Studien verbessert, erleichtert und/oder beschleunigt werden kann. Mittels einer Anonymisierung kann vorteilhaft eine Datenübertragung, insbesondere hinsichtlich einer Datensicherheit, verbessert werden. Vorteilhaft können dadurch sensible Daten, insbesondere Patientendaten, sicher übertragen werden. Ein Identifikator, insbesondere ein eindeutig einem Patienten zuordenbarer Identifikator, ist insbesondere als ein mit einer bestimmten Identität, beispielsweise einer Person und/oder eines Datensatzes, verknüpftes Merkmal zu einer eindeutigen Identifizierung der Identität, beispielsweise der Person und/oder des Datensatzes, ausgebildet. Insbesondere kann eine Sozialversicherungsnummer, eine Ausweisnummer, eine Passnummer, eine Krankenversicherungsnummer, eine Patientennummer und/oder eine Kundennummer einen Identifikator ausbilden

Des Weiteren weist die erfindungsgemäße Datenbankumgebung ein Identifikatormodul auf, welches auf Basis eines anonymisierten Patientendatensatzes einen eindeutigen anonymisierten Identifikator berechnet. Dadurch kann vorteilhaft eine Datenorganisation verbessert werden, wodurch insbesondere eine Effizienz gesteigert werden kann. Vorteilhaft ist der anonymisierte Identifikator unabhängig von eindeutig einer Person und/oder einem Erkennungsmerkmal zuordenbaren Komponenten, insbesondere indem ausschließlich bereits anonymisierte Daten zu einer Berechnung des anonymisieren Identifikators herangezogen werden. Ein anonymisierter Identifikator ist insbesondere als ein mit einem bestimmten Datensatz, beispielsweise einem Patientendatensatz, verknüpftes Merkmal zu einer eindeutigen Identifizierung des Datensatzes, insbesondere anonymisierten Patientendatensatzes, unter Beibehaltung einer Anonymität einer Identität, beispielsweise einer dem Datensatz, insbesondere anonymisierten Patientendatensatz, zugehörigen Person ausgebildet. Unter der Wendung, dass ein anonymisierter Identifikator "auf Basis eines anonymisierten Patientendatensatzes berechnet" wird soll insbesondere verstanden werden, dass zu der Berechnung des anonymisierten Identifikators Merkmale und/oder Eigenschaften des anonymisierten Patientendatensatzes verwendet werden. Insbesondere kann der anonymisierte Identifikator aus einer eindeutigen Signatur des anonymisierten Patientendatensatzes berechenbar sein. Die eindeutige Signatur kann insbesondere zumindest einen Teil der Patientendaten des anonymisierten Patientendatensatzes, beispielsweise eine Spitaleintrittszeit umfassen. Das Identifikatormodul kann vorteilhaft als ein separater Server innerhalb der Datenbankumgebung ausgebildet sein. Alternativ kann das Identifikatormodul vorteilhaft in einem Server der Datenbankumgebung, beispielsweise einen Datenbankserver der Datenbankumgebung und/oder einem Server des Anonymisierungsmoduls, integriert sein. Vorzugsweise ist jeder anonymisierten Patientendatenbank des Datenbankmoduls ein Identifikatormodul zugeordnet. Erfindungsgemäß verbleiben die anonymisierten Identifikatoren, insbesondere bis zu einer Löschung, dauerhaft in der Datenbankumgebung und/oder sind durch die Datentransporteinheit unversendbar. Erfindungsgemäß unterbindet das Beschränkungsmodul der Datentransporteinheit einen Versand von anonymisierten Identifikatoren.

Außerdem weist die Datenbankumgebung erfindungsgemäß ein Zuordnungsmodul auf, welches den anonymisierten Identifikator einem dem anonymisierten Patientendatensatz zugehörigen Patientendatensatz aus der Patientendatenbank zuordnet. Dadurch kann vorteilhaft eine, insbesondere intern auf die Datenbankumgebung beschränkte, Rückidentifikation eines anonymisierten Patientendatensatzes ermöglicht werden. Das Zuordnungsmodul kann vorteilhaft als ein separater Server innerhalb der Datenbankumgebung ausgebildet sein. Alternativ kann das Zuordnungsmodul vorteilhaft in einem Server der Datenbankumgebung, beispielsweise einen Datenbankserver der Datenbankumgebung, integriert sein. Vorzugsweise ist jeder Patientendatenbank des Datenbankmoduls und/oder jedem Identifikatormodul ein Zuordnungsmodul zugeordnet. Insbesondere ist das Zuordnungsmodul zwischen der Patientendatenbank und dem Identifikatormodul zwischengeschaltet. Insbesondere speichert das Datenbankmodul eine Tabelle ab, welche zumindest eine zugeordnete Liste von anonymisierten Identifikatoren und eindeutig zuordenbaren Identifikatoren und/oder Erkennungsmerkmalen aufweist. Vorzugsweise umfasst die Tabelle, insbesondere zusätzlich, zumindest einen einem anonymisierten Identifikator zugehörigen anonymisierten Patientendatensatz und/oder zumindest einen einem anonymisierten Patientendatensatz zugehörigen Patientendatensatz.

Weiterhin ist der anonymisierte Identifikator erfindungsgemäß als eine von dem Identifikatormodul berechnete, eindeutige Prüfsumme ausgebildet. Dadurch kann vorteilhaft eine Datenorganisation verbessert werden, wodurch insbesondere eine Effizienz gesteigert werden kann. Zudem kann vorteilhaft ein Datenschutz verbessert werden, insbesondere mittels einer hohen Sicherheit einer Prüfsumme gegenüber einer Entschlüsselung und/oder Rückberechnung. Vorteilhaft ist eine Berechnung und/oder Berechnungsmethode der Prüfsumme von außen, insbesondere von außerhalb des Identifikatormoduls, uneinsehbar, insbesondere nach außen geheim. Dadurch kann vorteilhaft eine Bereitschaft zur Anwendung und/oder Akzeptanz des Patientenrekrutierungssystems, insbesondere durch Spitäler, Patienten und/oder Ärzte erhöht werden, wodurch insbesondere eine Organisation von klinischen Studien verbessert, erleichtert und/oder beschleunigt werden kann. Die Prüfsumme kann insbesondere zumindest mittels zumindest einer Quersumme, zumindest einer Paritätsprüfung, zumindest einer "Fletcher's Checksum" Routine, zumindest einer zyklischen Redundanzprüfung, zumindest einer Moduloberechnung und/oder zumindest einem weiteren dem Fachmann sinnvoll erscheinenden Berechnungsverfahren ermittelt werden. Alternativ oder zusätzlich kann eine Implementierung der Prüfsumme zumindest einen "Universal Unique Identifier" (UUID) und/oder zumindest einen serialisierten String umfassen.

Ferner wird ein Verschlüsselungsmodul der Datenbankumgebung, welches zumindest die, zumindest einem anonymisierten Patientendatensatz zugehörigen, anonymisierten Identifikatoren und/oder eine Tabelle des Datenbankmoduls verschlüsselt, vorgeschlagen. Vorteilhaft kann eine Sicherheit gegenüber einem unautorisierten Zugriff auf sensible Daten verbessert werden. Dadurch kann vorteilhaft ein Datenschutz verbessert werden, insbesondere mittels einer zusätzlichen Verschlüsselung der anonymisierten Identifikatoren und/oder der Tabelle auf der Datenbankumgebungsseite. Dadurch kann vorteilhaft eine Bereitschaft zur Anwendung und/oder Akzeptanz des Patientenrekrutierungssystems, insbesondere durch Spitäler, Patienten und/oder Ärzte erhöht werden, wodurch insbesondere eine Organisation von klinischen Studien verbessert, erleichtert und/oder beschleunigt werden kann. Das Verschlüsselungsmodul kann vorteilhaft als ein separater Server innerhalb der Datenbankumgebung ausgebildet sein. Alternativ kann das Verschlüsselungsmodul vorteilhaft in einem Server der Datenbankumgebung, beispielsweise dem Zuordnungsmodul der Datenbankumgebung und/oder einem Datenbankserver der Datenbankumgebung integriert sein. Vorzugsweise ist jeder Patientendatenbank und/oder jeder Tabelle des Datenbankmoduls ein Verschlüsselungsmodul zugeordnet. Insbesondere ist das Verschlüsselungsmodul zwischen dem Identifikatormodul und dem Zuordnungsmodul zwischengeschaltet. Vorzugsweise verschlüsselt das Verschlüsselungsmodul die anonymisierten Identifikatoren und/oder die Tabelle zumindest mittels eines, insbesondere dem Stand der Technik hinreichenden, symmetrischen Verschlüsselungsverfahrens, wie beispielsweise AES, IDEA, Blowfish oder mittels eines RSA Verfahrens.

Des Weiteren zieht das Identifikatormodul erfindungsgemäß zu einer Berechnung des anonymisierten Identifikators zumindest eine Uhrzeit, insbesondere einen Spitaleintrittszeitpunkt, und zumindest eine, insbesondere der Uhrzeit, vorzugsweise dem Spitaleintrittszeitpunkt, zugeordnete, medizinische Diagnose, zumindest eine, insbesondere der Uhrzeit, vorzugsweise dem Spitaleintrittszeitpunkt, zugeordnete, Dosierung, insbesondere einer Medikation, zumindest einen, insbesondere der Uhrzeit, vorzugsweise dem Spitaleintrittszeitpunkt, zugeordneten, Laborwert, und/oder zumindest eine, insbesondere der Uhrzeit, vorzugsweise dem Spitaleintrittszeitpunkt, zugeordnete, Medikation, insbesondere einen Medikationsverlauf, heran. Dadurch kann vorteilhaft eine Berechnung eines eindeutigen anonymisierten Identifikators ermöglicht werden. Zudem kann vorteilhaft ein, insbesondere zeitlich veränderlicher anonymisierter Identifikator, welcher insbesondere verschieden ist nach jeder Aktualisierung eines Patientendatensatzes, ermöglicht werden. Es ist vorstellbar, dass das Identifikatormodul zumindest einen Text und/oder zumindest einen Zahlenwert des anonymisierten Patientendatensatzes zu der Berechnung des anonymisierten Identifikators heranzieht. Die Uhrzeit kann insbesondere einen Spitaleintrittszeitpunkt, einen Spitalaustrittszeitpunkt, einen Diagnosezeitpunkt, einen Behandlungszeitpunkt und/oder einen Zeitpunkt einer Verabreichung und/oder Verschreibung einer Medikation umfassen.

Außerdem ordnet das Identifikatormodul erfindungsgemäß bei einer Aktualisierung, eines bereits in einer anonymisierten Patientendatenbank des Datenbankmoduls vorhandenen, bereits, insbesondere von dem Zuordnungsmodul, mit einem anonymisierten Identifikator versehenen anonymisierten Patientendatensatzes, insbesondere bei einer Aktualisierung des zugehörigen Behandlungsverlaufs, dem zugehörigen anonymisierten Patientendatensatz einen eindeutigen aktualisierten auf Basis eines zugehörigen aktualisierten anonymisierten Patientendatensatzes berechneten, anonymisierten Identifikator, welcher verschieden ist von dem anonymisierten Identifikator, zu. Dadurch kann vorteilhaft einem Patientendatensatz ein zeitlich variabler Identifikator zugeordnet werden. Dadurch kann vorteilhaft eine unautorisierte Rückidentifikation erschwert werden, wodurch insbesondere ein Datenschutz verbessert werden kann. Insbesondere können einem Patientendatensatz in der Tabelle einer oder zwei oder mehr als zwei anonymisierte Identifikatoren zugeordnet sein. Insbesondere ist einem eindeutig zuordenbaren Identifikator und/oder Erkennungsmerkmal in der Tabelle zumindest ein aktualisierter anonymisierter Identifikator und ein ursprünglicher anonymisierter Identifikator, welcher dem Patientendatensatz vor einer Aktualisierung zugeordnet wurde, zuordenbar. Zudem ist denkbar, dass die Zuordnungseinheit dem aktualisierten anonymisierten Identifikator zumindest einen aktualisierten anonymisierten Patientendatensatz und zumindest einen ursprünglichen anonymisierten Patientendatensatz, welcher insbesondere dem anonymisierten Patientendatensatz vor der Aktualisierung entspricht, zuordnet. Dadurch kann vorteilhaft eine ursprüngliche Zuordnung vor der Aktualisierung nachvollziehbar bleiben. Alternativ überschreibt das Zuordnungsmodul bei einer Aktualisierung den ursprünglichen anonymisierten Identifikator mit dem aktualisierten anonymisierten Identifikator. Dadurch kann vorteilhaft ein Datenschutz verbessert werden.

Zudem überträgt die Datentransporteinheit erfindungsgemäß bei einer Übertragung eines aktualisierten anonymisierten Patientendatensatzes von der Datenbankumgebung zu dem Patientenrekrutierungsserver einen ursprünglichen anonymisierten Patientendatensatz, welcher insbesondere dem anonymisierten Patientendatensatz vor einer Aktualisierung entspricht, mit. Dadurch kann vorteilhaft eine Aktualisierung eines anonymisierten Patientendatensatzes auf dem Patientenrekrutierungsserver ermöglicht werden, wodurch vorteilhaft Doppel- oder Mehrfacheinträge von anonymisierten Patientendatensätzen auf einer Patientenrekrutierungsserverseite, insbesondere in einer Patientenrekrutierungsdatenbank des Patientenrekrutierungsservers, vermieden werden können. Vorzugsweise überträgt die Datentransporteinheit den aktualisierten anonymisierten Patientendatensatz und den ursprünglichen anonymisierten Patientendatensatz in einer Form, welche sie dem Patientenrekrutierungsserver als zusammengehörig kennzeichnet, beispielsweise in zumindest einem gemeinsamen Datenpaket und/oder einem gemeinsamen Bündel an Datenpaketen. Insbesondere ist die Übertragung und/oder Aktualisierung von anonymisierten Patientendatensätzen frei von jeglichen Identifikatoren. Erfindungsgemäß weist der Patientenrekrutierungsserver ein Identifikatormodul auf, welches dazu vorgesehen ist, einen anonymisierten Identifikator, insbesondere auf Basis einer Prüfsumme, einem von der Datentransporteinheit empfangenen anonymisierten Patientendatensatz zuzuordnen. Vorzugsweise ist der Identifikator, insbesondere die Prüfsumme und/oder eine Prüfsummenberechnungsmethode des Identifikatormoduls des Patientenrekrutierungsservers verschieden von der Prüfsumme und/oder der Prüfsummenberechnungsmethode des Identifikatormoduls der Datenbankumgebung. Insbesondere ist eine Prüfsummenberechnung innerhalb der Datenbankumgebung und eine Prüfsummenberechnung innerhalb des Patientenrekrutierungsservers unabhängig voneinander. Mittels einer erfindungsgemäßen Zuordnung eines Identifikators auf der Patientenrekrutierungsserverseite wird ein Auffinden zumindest einer Übereinstimmung von identischen anonymisierten Patientendatensätzen beschleunigt.

Ferner umfasst der Patientenrekrutierungsserver erfindungsgemäß ein Datensatzabgleichmodul, welches dazu vorgesehen ist empfangene, anonymisierte Patientendatensätze mit anonymisierten Patientendatensätzen in einer Patientenrekrutierungsdatenbank des Patientenrekrutierungsservers abzugleichen und bei einer Übereinstimmung zumindest eines anonymisierten Patientendatensatzes einen zugehörigen Datenbankeintrag der Patientenrekrutierungsdatenbank mit einem zugehörigen aktualisierten anonymisierten Patientendatensatz überschreibt. Dadurch kann vorteilhaft eine effektive Aktualisierung eines anonymisierten Patientendatensatzes auf dem Patientenrekrutierungsserver ermöglicht werden, wodurch vorteilhaft Doppel- oder Mehrfacheinträge von anonymisierten Patientendatensätzen auf einer Patientenrekrutierungsserverseite, insbesondere in der Patientenrekrutierungsdatenbank des Patientenrekrutierungsservers, vermieden werden können. Vorteilhaft ist die Aktualisierung von anonymisierten Patientendatensätzen auf der Patientenrekrutierungsserverseite unabhängig von jeglichen, insbesondere auf der Datenbankumgebungsseite berechneten und/oder vergebenen Identifikatoren. Dadurch kann vorteilhaft ein Datenschutz verbessert werden. Erfindungsgemäß erkennt das Datensatzablgeichsmodul des Patientenrekrutierungsservers bei einem Empfang eines bereits vorhandenen anonymisierten Patientendatensatzes eine Übereinstimmung der anonymisierten Patientendatensätze und veranlasst ein Überschreiben des vorhandenen anonymisierten Patientendatensatzes mit dem zusammen empfangenen aktualisierten anonymisierten Patientendatensatz in der Patientenrekrutierungsdatenbank. Das Datensatzabgleichsmodul kann vorteilhaft als ein separater mit dem Patientenrekrutierungsserver in Kommunikation stehender Server innerhalb eines gemeinsamen Intranets ausgebildet sein. Alternativ kann das Datensatzabgleichsmodul vorteilhaft in dem Patientenrekrutierungsserver integriert sein. Vorzugsweise ist das Datensatzabgleichsmodul zwischen dem Empfänger der Datentransporteinheit und der Patientenrekrutierungsdatenbank des Patientenrekrutierungsservers zwischengeschaltet. Die Patientenrekrutierungsdatenbank ist insbesondere dazu vorgesehen, von der Datentransporteinheit empfangene anonymisierte Patientendatensätze abzuspeichern und für eine Durchforstung mittels des Patientenrekrutierungsmoduls der Datenbankumgebung freizugeben und/oder bereitzuhalten. Ein Datenbankeintrag der Patientenrekrutierungsdatenbank umfasst erfindungsgemäß zumindest einen aktualisierten, anonymisierten Patientendatensatz und/oder zumindest einen, insbesondere von dem Identifikatormodul des Patientenrekrutierungsservers berechneten anonymisierten Identifikator.

Außerdem wird vorgeschlagen, dass der Patientenrekrutierungsserver zumindest eine Internetschnittstelle, insbesondere zu einer zentralen Cloudinstanz eines Betreibers eines Patientenrekrutierungsservers, zumindest zu einem Empfang von zumindest einem Rekrutierungsmerkmal aufweist, wobei eine Verbindung der Internetschnittstelle zu der zentralen Cloudinstanz vorzugsweise als eine von dem Patientenrekrutierungsserver ausgehende Outbound Verbindung ausgebildet ist. Dadurch kann vorteilhaft ein Auffinden von geeigneten Probanden für eine klinische Studie verbessert und/oder beschleunigt werden. Vorteilhaft können Rekrutierungsmerkmale einer Vielzahl unterschiedlichster Studien auf den Patientenrekrutierungsserver geladen werden. Es ist beispielsweise vorstellbar, dass ein Betreiber eines Patientenrekrutierungsservers mittels der Internetschnittstelle eine Datenbank an Rekrutierungsmerkmalen und/oder klinischen Studien durchforsten und für ihn interessante Studien auswählen kann. Alternativ oder zusätzlich ist vorstellbar, dass ein Organisator von klinischen Studien Rekrutierungsmerkmale an zumindest einen Patientenrekrutierungsserver versenden kann. Insbesondere ist vorstellbar, dass mittels der Internetschnittstelle dem Organisator von klinischen Studien eine Anzahl an von dem Patientenrekrutierungsserver anhand übermittelter Rekrutierungsmerkmale bestimmter anonymisierter Patientendatensätze, insbesondere Probanden, angezeigt wird. Die Internetschnittstelle kann insbesondere dazu vorgesehen sein eine Verbindung mit einer Cloud und/oder mit einem, insbesondere virtuellen, Server auszubilden, welche/welcher vorzugsweise mit einer Mehrzahl an Patientenrekrutierungsservern an verschiedenen Standorten kommuniziert und vorzugsweise aus dem Internet anwählbar ist. Der Patientenrekrutierungsserver weist insbesondere ein Beschränkungsmodul auf, welches einen Datentransfer von anonymisierten Patientendatensätzen an die Internetschnittstelle unterbindet. Vorzugsweise kommuniziert der Patientenrekrutierungsserver mit der Internetschnittstelle ausschließlich mittels einer von dem Patientenrekrutierungsserver ausgehenden Outbound Verbindung.

Weiterhin wird vorgeschlagen, dass ein Patientenrekrutierungsmodul der Datenbankumgebung mittels der Outbound Verbindung der Datentransporteinheit zumindest einen anonymisierten Patientendatensatz, welchem mittels dem Patientenrekrutierungsmodul des Patientenrekrutierungsservers zumindest ein Rekrutierungsmerkmal, insbesondere zumindest eine Kombination von Rekrutierungsmerkmalen, zugeordnet ist und/oder zumindest ein Rekrutierungsmerkmal, insbesondere zumindest eine Kombination von Rekrutierungsmerkmalen, von dem Patientenrekrutierungsserver anfordert. Dadurch kann vorteilhaft eine sichere Datenübertragung, insbesondere einer Auswahl von auf der Patientenrekrutierungsserverseite aufgefundenen passenden Kandidaten zurück an die Datenbankumgebung ermöglicht werden, wodurch insbesondere eine Datensicherheit verbessert werden kann. Vorteilhaft können dadurch auf der Patientenrekrutierungsserverseite aufgefundene passende Kandidaten unabhängig von Identifikatoren auf die Datenbankumgebungsseite rückübertragen werden, insbesondere mit einem Ziel einer Rückidentifikation auf der, insbesondere besonders geschützten, Datenbankumgebungsseite. Dadurch kann vorteilhaft eine hinsichtlich eines Datenschutzes besonders sichere Rückidentifikation ermöglicht werden. Vorzugsweise erfolgt ein Anfordern von anonymisierten Patientendatensätzen und/oder Rekrutierungsmerkmalen von dem Patientenrekrutierungsmodul der Datenbankumgebung automatisch und/oder in regelmäßigen Abständen. Unter einem "passenden Kandidat" soll insbesondere ein anonymisierter Patientendatensatz verstanden werden, welchem von dem Patientenrekrutierungsmodul des Patientenrekrutierungsservers ein, insbesondere zu einer klinischen Studie passendes, Rekrutierungsmerkmal erfolgreich zugeordnet werden konnte. Insbesondere fordert das Patientenrekrutierungsmodul der Datenbankumgebung zusätzlich zu zumindest einem passenden Kandidaten zumindest eine zugehörige klinische Studie an, wodurch vorteilhaft eine Zuordnung des passenden Kandidaten zu einer klinischen Studie auf der Datenbankumgebungsseite ermöglicht werden kann. Alternativ oder zusätzlich fordert das Patientenrekrutierungsmodul der Datenbankumgebung zumindest ein, insbesondere einer klinischen Studie zugehöriges, Rekrutierungsmerkmal an, woraufhin die Datentransporteinheit eine, insbesondere gemeinsame, Rückübertragung des zumindest einen Rekrutierungsmerkmals der klinischen Studie zusammen mit allen anonymisierten Patientendatensätzen denen das zumindest eine Rekrutierungsmerkmal zugeordnet werden konnte von dem Patientenrekrutierungsserver an die Datenbankumgebung vornimmt. Insbesondere ist die Datentransporteinheit dazu vorgesehen in Folge einer Anforderung zumindest eines anonymisierten Patientendatensatzes, welchem das Patientenrekrutierungsmodul des Patientenrekrutierungsservers zumindest ein, insbesondere zu zumindest einer klinischen Studie gehöriges, Rekrutierungsmerkmal, insbesondere erfolgreich, zugeordnet hat, zumindest den zugeordneten anonymisierten Patientendatensatz zu übertragen. Vorzugsweise überträgt die Datentransporteinheit einen zumindest einem Rekrutierungsmerkmal zugeordneten anonymisierten Patientendatensatz zusammen mit dem zumindest einem Rekrutierungsmerkmal, insbesondere in einem Datenpaket und/oder einem gemeinsamen Bündel an Datenpaketen. Das Patientenrekrutierungsmodul des Patientenrekrutierungsservers kann vorteilhaft als ein separater Server, welcher mit dem Patientenrekrutierungsserver, vorzugsweise innerhalb eines gemeinsamen Intranets, kommuniziert ausgebildet sein. Alternativ kann das Patientenrekrutierungsmodul des Patientenrekrutierungsservers vorteilhaft in dem Patientenrekrutierungsserver integriert sein. Darunter, dass Daten "mittels einer Outbound Verbindung angefordert" werden soll insbesondere verstanden werden, dass vor einer Übertragung der Daten eine Empfangsseite eine, zumindest eine Vorgaben an die zu versendenden Daten enthaltende Sendeaufforderung an die Senderseite stellt, welche daraufhin ausschließlich Daten, welche die Vorgaben einhalten und/oder erfüllen versendet und/oder versenden kann.

Außerdem wird vorgeschlagen, dass ein Rückidentifikationsmodul der Datenbankumgebung bei einem Empfang eines durch das Patientenrekrutierungsmodul der Datenbankumgebung angeforderten anonymisierten Patientendatensatzes eine Rückidentifizierung des anonymisierten Patientendatensatzes durchführt, insbesondere mittels einer Neuberechnung und eines Abgleichs zumindest eines anonymisierten Identifikators. Dadurch kann vorteilhaft eine Datensicherheit erhöht werden, insbesondere indem eine Rückidentifikation ausschließlich in der besonders gesicherten Datenbankumgebung erfolgen kann. Vorteilhaft kann ein Datenschutz verbessert werden. Dadurch kann vorteilhaft eine Bereitschaft zur Anwendung und/oder Akzeptanz des Patientenrekrutierungssystems, insbesondere durch Spitäler, Patienten und/oder Ärzte erhöht werden, wodurch insbesondere eine Organisation von klinischen Studien verbessert, erleichtert und/oder beschleunigt werden kann. Zu einer Rückidentifizierung eines empfangenen anonymisierten Patientendatensatzes berechnet das Rückidentifikationsmodul einen anonymisierten Identifikator auf Basis des empfangenen anonymisierten Patientendatensatzes. Anschließend gleicht das Zuordnungsmodul den berechneten anonymisierten Identifikator des empfangenen anonymisierten Patientendatensatzes mit in der Tabelle des Datenbankmoduls enthaltenen anonymisierten Identifikatoren ab. Bei einer Übereinstimmung eines anonymisierten Identifikators der Tabelle des Datenbankmoduls mit einem berechneten anonymisierten Identifikator des empfangenen anonymisierten Patientendatensatzes identifiziert das Zuordnungsmodul den zugehörigen Patientendatensatz, vorzugsweise den zugehörigen Identifikator des Patientendatensatzes in der Patientendatenbank, welcher insbesondere zumindest ein eindeutig einer Person zuordenbares Erkennungsmerkmal enthält. Es ist vorstellbar, dass das Zuordnungsmodul den Patientendatensatz, vorzugsweise den Patientenidentifikator, an ein Anzeigemodul der Datenbankumgebung übergibt. Das Anzeigemodul ist dazu vorgesehen einem autorisierten Bediener den Patientendatensatz zumindest teilweise visuell auszugeben, beispielsweise mittels eines Bildschirms und/oder eines Ausdrucks. Vorzugsweise ist zumindest ein Zugriff auf das Anzeigemodul und/oder ein Zugang zu einem Raum in dem sich das Anzeigemodul befindet zugangsbeschränkt und/oder passwortgeschützt.

Insbesondere ist vorstellbar, dass das Datenbankmodul, das Patientenrekrutierungsmodul der Datenbankumgebung, das Beschränkungsmodul der Datentransporteinheit, das Anonymisierungsmodul, das Identifikatormodul, das Zuordnungsmodul, das Verschlüsselungsmodul, das Rückidentifikationsmodul und/oder das Anzeigemodul zumindest teilweise Teil eines gemeinsamen Computersystems und/oder zumindest teilweise Teil eines gemeinsamen Servers sind.

Zudem wird ein Verfahren zur Rekrutierung von Patienten, insbesondere für klinische Studien, mittels eines Patientenrekrutierungssystems mit zumindest einer Datenbankumgebung, die zumindest ein Datenbankmodul aufweist, welches dazu vorgesehen ist zumindest Patientendatensätze mit zumindest einem Behandlungsverlauf zumindest eines Patienten in einer Patientendatenbank abzuspeichern, mit zumindest einem Patientenrekrutierungsserver, welcher zumindest ein Patientenrekrutierungsmodul aufweist, welches dazu vorgesehen ist Behandlungsverläufe zu durchsuchen und zumindest mit zumindest einem Rekrutierungsmerkmal abzugleichen und mit einer Datentransporteinheit zu einem Datentransfer, insbesondere zumindest eines Behandlungsverlaufs, zwischen der Datenbankumgebung und dem Patientenrekrutierungsserver, wobei der Datentransfer von einem Beschränkungsmodul der Datentransporteinheit auf von der Datenbankumgebung ausgehende Outbound Verbindungen beschränkt wird, vorgeschlagen. Dadurch kann vorteilhaft eine Datenübertragung, insbesondere hinsichtlich einer Datensicherheit, verbessert werden. Vorteilhaft können dadurch sensible Daten, insbesondere Patientendaten, sicher übertragen werden. Insbesondere ist die Datentransporteinheit auf der Seite der Datenbankumgebung frei von Inbound Ports und weist ausschließlich Outbound Ports auf, wodurch vorteilhaft sichergestellt werden kann, dass Verbindungen zwischen der Datenbankumgebung und dem Patientenrekrutierungsserver immer von der Seite der Datenbankumgebung initiiert werden. Dadurch kann vorteilhaft erreicht werden, dass der durch die Datentransporteinheit übermittelte Datentransfer für die Seite der Datenbankumgebung vollständig transparent ist. Zudem kann vorteilhaft ein unautorisierter Zugriff auf die Datenbankumgebung mittels der Datentransporteinheit, beispielsweise durch "hacking", unterbunden werden.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Ansicht eines Patientenrekrutierungssystems,
- Fig. 2: ein Ablaufdiagramm für ein Verfahren zur Rekrutierung von Patienten,
- Fig. 3: ein Ablaufdiagramm eines Teils des Verfahrens,
- Fig. 4: ein Ablaufdiagramm eines weiteren Teils des Verfahrens und
- Fig. 5: ein Ablaufdiagramm eines zusätzlichen weiteren Teils des Verfahrens.

### Beschreibung der Ausführungsbeispiele

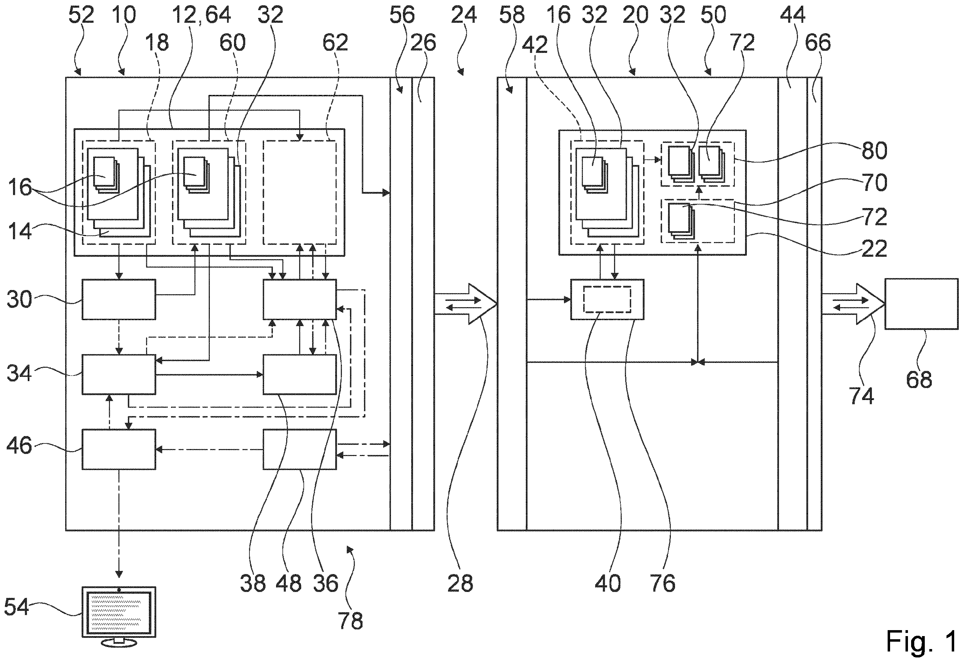

Fig. 1 zeigt ein Patientenrekrutierungssystem. Das Patientenrekrutierungssystem ist dazu vorgesehen Patienten für klinische Studien zu rekrutieren. Das Patientenrekrutierungssystem weist eine Datenbankumgebung 10 auf. Die Datenbankumgebung 10 weist ein Datenbankmodul 12 auf. Das Datenbankmodul ist als ein Datenbankserver 64 ausgebildet. Das Datenbankmodul 12 ist dazu vorgesehen Daten und/oder Datensätze, insbesondere in Datenbanken und/oder Tabellen, abzuspeichern. Das Datenbankmodul 12 weist eine Patientendatenbank 18 auf. In der Patientendatenbank 18 sind Patientendatensätze 14 abspeicherbar. Ein Patientendatensatz 14 weist zumindest einen Behandlungsverlauf 16 eines Patienten auf. Das Datenbankmodul 12 weist eine anonymisierte Patientendatenbank 60 auf. In der anonymisieren Patientendatenbank 60 sind anonymisierte Patientendatensätze 32 abspeicherbar. Ein anonymisierter Patientendatensatz 32 weist zumindest einen Behandlungsverlauf 16 eines anonymisierten Patienten auf. Das Datenbankmodul 12 weist eine Tabelle 62 auf. In der Tabelle 62 sind Patientendatensätze 14 abspeicherbar. In der Tabelle 62 sind anonymisierte Patientendatensätze 32 abspeicherbar. In der Tabelle 62 sind Identifikatoren abspeicherbar. In der Tabelle 62 sind anonymisierte Identifikatoren abspeicherbar.

Das Patientenrekrutierungssystem weist eine Datentransporteinheit 24 auf. Die Datentransporteinheit 24 ist zu einem Datentransfer eines anonymisierten Patientendatensatzes 32 vorgesehen. Die Datentransporteinheit 24 weist einen Sender 56 auf. Der Sender 56 ist auf einer Datenbankumgebungsseite 52 des Patientenrekrutierungssystems angeordnet. Die Datentransporteinheit 24 weist einen Empfänger 58 auf. Der Empfänger 58 ist auf einer Patientenrekrutierungsserverseite 50 des Patientenrekrutierungssystems angeordnet. Die Datentransporteinheit 24 steuert einen Datentransfer zwischen der Datenbankumgebung 10 und dem Patientenrekrutierungsserver 20. Die Datentransporteinheit 24 übermittelt Daten zwischen der Datenbankumgebung 10 und dem Patientenrekrutierungsserver 20. Die Datentransporteinheit 24 ermöglicht einen Datentransport von der Datenbankumgebung 10 zu dem Patientenrekrutierungsserver 20. Die Datentransporteinheit 24 ermöglicht einen Datentransport von dem Patientenrekrutierungsserver 20 zu der Datenbankumgebung 10. Die Datentransporteinheit 24 weist ein Beschränkungsmodul 26 auf. Das Beschränkungsmodul 26 beschränkt den Datentransfer auf von der Datenbankumgebung 10 ausgehende Outbound Verbindungen 28. Die Outbound Verbindung 28 erlaubt ausschließlich von der Datenbankumgebung 10 initiierten Datentransport. Der Sender 56 steuert den Datentransport eingehender und ausgehender Daten.

Die Datenbankumgebung 10 weist ein Anonymisierungsmodul 30 auf. Das Anonymisierungsmodul 30 ist dazu vorgesehen, Patientendatensätze 14 in anonymisierte Patientendatensätze 32 umzuwandeln. Das Anonymisierungsmodul 30 empfängt Patientendatensätze 14 von der Patientendatenbank 18. Das Anonymisierungsmodul 30 entfernt Erkennungsmerkmale aus den Patientendatensätzen 14. Durch das Anonymisierungsmodul 30 anonymisierte Patientendatensätze 32 sind frei sind von eindeutig einem Patienten zuordenbaren Identifikatoren und/oder Erkennungsmerkmalen. Das Anonymisierungsmodul 30 transferiert anonymisierte Patientendatensätze 32 zu der anonymisierten Patientendatenbank 60.

Die Datenbankumgebung 10 weist ein Identifikatormodul 34 auf. Das Identifikatormodul 34 empfängt anonymisierte Patientendatensätze 32 von der anonymisierten Patientendatenbank 60 und/oder von einem Rückidentifikationsmodul 46 der Datenbankumgebung 10. Alternativ ist vorstellbar, dass das Anonymisierungsmodul 30 die anonymisierten Patientendatensätze 32 direkt an das Identifikatormodul 34 weiterleitet. Das Identifikatormodul 34 berechnet erfindungsgemäß auf Basis eines anonymisierten Patientendatensatzes 32 einen eindeutigen anonymisierten Identifikator. Das Identifikatormodul 34 zieht zu einer Berechnung des anonymisierten Identifikators zumindest eine Uhrzeit, zumindest eine medizinische Diagnose, zumindest eine Dosierung, zumindest einen Laborwert, und/oder zumindest eine Medikation heran. Der anonymisierte Identifikator ist als eine eindeutige Prüfsumme ausgebildet. Das Identifikatormodul 34 berechnet die Prüfsumme. Der erfindungsgemäß anonymisierte Identifikator ist zeitlich veränderlich ausgebildet, bei einer Veränderung eines anonymisierten Patientendatensatzes 32 verändert sich erfindungsgemäß der anonymisierte Identifikator. Das Identifikatormodul 34 transferiert den anonymisierten Identifikator und den zugehörigen anonymisierten Patientendatensatz 32 an ein Verschlüsselungsmodul 38 der Datenbankumgebung 10.

Das Identifikatormodul 34 weist bei einer Aktualisierung eines bereits in der anonymisieren Patientendatenbank 60 vorhandenen, bereits mit einem anonymisierten Identifikator versehenen anonymisierten Patientendatensatzes 32 dem zugehörigen anonymisierten Patientendatensatz 32 einen eindeutigen aktualisierten anonymisierten Identifikator zu. Das Identifikatormodul 34 berechnet den aktualisierten anonymisierten Identifikator auf Basis des zugehörigen aktualisierten anonymisierten Patientendatensatzes 32. Der aktualisierte anonymisierte Identifikator ist verschieden von dem anonymisierten Identifikator. Die Datentransporteinheit 24 überträgt bei einer Übertragung eines aktualisierten anonymisierten Patientendatensatzes 32 von der Datenbankumgebung 10 zu dem Patientenrekrutierungsserver 20 einen ursprünglichen anonymisierten Patientendatensatz 32 mit.

Die Datenbankumgebung 10 weist das Verschlüsselungsmodul 38 auf. Das Verschlüsselungsmodul 38 verschlüsselt die, zumindest einem anonymisierten Patientendatensatz 32 zugehörigen, anonymisierten Identifikatoren. Das Verschlüsselungsmodul 38 empfängt anonymisierte Identifikatoren von dem Identifikatormodul 34. Zusätzlich kann das Verschlüsselungsmodul 38 dazu vorgesehen sein anonymisierte Identifikatoren zu entschlüsseln. Alternativ ist vorstellbar, dass das Patientenrekrutierungssystem frei ist von einem Verschlüsselungsmodul 38 und das Identifikatormodul 34 die anonymisierten Identifikatoren direkt zu einem Zuordnungsmodul 36 der Datenbankumgebung 10 transferiert (siehe gestrichelter Pfad in Figur 1).

Die Datenbankumgebung 10 weist das Zuordnungsmodul 36 auf. Das Zuordnungsmodul 36 empfängt verschlüsselte oder unverschlüsselte anonymisierte Identifikatoren von dem Verschlüsselungsmodul 38 oder dem Identifikatormodul 34. Das Zuordnungsmodul 36 empfängt Patientendatensätze 14 von der Patientendatenbank 18. Das Zuordnungsmodul 36 empfängt anonymisierte Patientendatensätze 32 von der anonymisierten Patientendatenbank 60. Das Zuordnungsmodul 36 ordnet den anonymisierten Identifikator einem dem anonymisierten Patientendatensatz 32 zugehörigen Patientendatensatz 14 aus der Patientendatenbank 18 zu. Das Zuordnungsmodul 36 speichert eine Zuordnung von Patientendatensätzen 14, anonymisierten Patientendatensätzen 32 und anonymisierten Identifikatoren in der Tabelle 62 ab. Alternativ ist vorstellbar, dass das Zuordnungsmodul 36 nur eine Zuordnung von Patientendatensätzen 14 und anonymisierten Identifikatoren in der Tabelle 62 abspeichert.

Das Patientenrekrutierungssystem weist einen Patientenrekrutierungsserver 20 auf. Der Patientenrekrutierungsserver 20 weist ein Patientenrekrutierungsmodul 22 auf. Das Patientenrekrutierungsmodul 22 weist eine Rekrutierungsmerkmalsdatenbank 70 auf. In der Rekrutierungsmerkmalsdatenbank 70 sind Rekrutierungsmerkmale 72 abspeicherbar. Das Patientenrekrutierungsmodul 22 ist dazu vorgesehen Datensätze, insbesondere Behandlungsverläufe 16, zu durchsuchen. Das Patientenrekrutierungsmodul 22 ist dazu vorgesehen anonymisierte Patientendatensätze 32 mit zumindest einem Rekrutierungsmerkmal 72 abzugleichen. Das Patientenrekrutierungsmodul 22 weist eine Kandidatenliste 80 auf. In der Kandidatenliste 80 sind Rekrutierungsmerkmalen 72 zugeordnete anonymisierte Patientendatensätze 32 abspeicherbar. In der Kandidatenliste 80 sind Rekrutierungsmerkmale 72 zu anonymisierten Patientendatensätzen 32 zugeordnet abspeicherbar.

Der Patientenrekrutierungsserver 20 weist ein Datenempfangsmodul 76 auf. Das Datenempfangsmodul 76 ist dazu vorgesehen anonymisierte Patientendatensätze 32 von der Datentransporteinheit 24 zu empfangen. Das Patientenrekrutierungsmodul 22 weist eine Patientenrekrutierungsdatenbank 42 auf. In der Patientenrekrutierungsdatenbank 42 sind anonymisierte Patientendatensätze 32 abspeicherbar. Das Datenempfangsmodul 76 ist dazu vorgesehen empfangene anonymisierte Patientendatensätze 32 in der Patientenrekrutierungsdatenbank 42 abzuspeichern. Der Patientenrekrutierungsserver 20 umfasst ein Datensatzabgleichmodul 40. Das Datensatzabgleichmodul 40 ist dazu vorgesehen, empfangene, anonymisierte Patientendatensätze 32 mit anonymisierten Patientendatensätzen 32 in der Patientenrekrutierungsdatenbank 42 abzugleichen. Das Datensatzabgleichmodul 40 ist dazu vorgesehen, bei einer Übereinstimmung eines anonymisierten Patientendatensatzes 32 einen zugehörigen Datenbankeintrag der Patientenrekrutierungsdatenbank 42 mit einem zugehörigen aktualisierten anonymisierten Patientendatensatz 32 zu überschreiben.

Der Patientenrekrutierungsserver 20 weist eine Internetschnittstelle 44 auf. Die Internetschnittstelle weist ein Beschränkungsmodul 66 auf. Das Beschränkungsmodul 66 ist als eine Firewall ausgebildet. Die Internetschnittstelle 44 dient zu einem Empfang von zumindest einem Rekrutierungsmerkmal 72. Die Internetschnittstelle 44 ist dazu vorgesehen eine elektronische Verbindung mit einer Cloud 68 herzustellen. Vorzugsweise ist die Internetschnittstelle 44 beschränkt auf die elektronische Verbindung mit der Cloud 68. Die Cloud 68 ist als eine zentrale Cloud, insbesondere eines Betreibers des Patientenrekrutierungssystems, ausgebildet. Das Beschränkungsmodul 66 beschränkt die Kommunikation zwischen dem Patientenrekrutierungsserver 20 und der Cloud auf eine Outbound Verbindung des Patientenrekrutierungsservers 20. Die Internetschnittstelle 44 kommuniziert mit der Cloud 68 über die Outbound Verbindung 74 des Patientenrekrutierungsservers 20. Die Outbound Verbindung 74 des Patientenrekrutierungsservers 20 besteht zwischen dem Patientenrekrutierungsserver 20 und der Cloud 68. Die Outbound Verbindung 74 des Patientenrekrutierungsservers 20 ist ausschließlich von dem Patientenrekrutierungsserver 20 ausgehend.

Die Datenbankumgebung 10 weist eine Rückidentifikationseinheit 78 auf. Die Rückidentifikationseinheit 78 ist teilweise einstückig mit der Datenbankumgebung 10 ausgebildet. Darunter, dass eine Einheit und eine Umgebung "teilweise einstückig" ausgebildet sind, soll insbesondere verstanden werden, dass die Einheit und die Umgebung zumindest ein, insbesondere zumindest zwei, vorteilhaft zumindest drei gemeinsame Elemente aufweisen, die Bestandteil, insbesondere funktionell wichtiger Bestandteil, beider Einheiten sind. In der Figur 1 ist ein Kommunikationspfad der Rückidentifikationseinheit 78 bei einer Rückidentifizierung eines anonymisierten Patientendatensatzes 32, welchem zumindest ein Rekrutierungsmerkmal 72 auf der Patientenrekrutierungsserverseite 50 zugeordnet wurde mit Strichpunktlinien gekennzeichnet.

Die Datenbankumgebung 10 weist ein Patientenrekrutierungsmodul 48 auf. Das Patientenrekrutierungsmodul 48 der Datenbankumgebung 10 ist zumindest dazu vorgesehen einen anonymisierten Patientendatensatz 32 dem ein Rekrutierungsmerkmal 72 zugeordnet ist von dem Patientenrekrutierungsserver 20 anzufordern. Das Patientenrekrutierungsmodul 48 der Datenbankumgebung 10 fordert einen anonymisierten Patientendatensatz 32 dem ein Rekrutierungsmerkmal 72 zugeordnet ist mittels der Outbound Verbindung 28 an. Die Datenbankumgebung 10 weist ein Rückidentifikationsmodul 46 auf. Das Rückidentifikationsmodul 46 ist dazu vorgesehen anonymisierte Patientendatensätze 32 denen ein Rekrutierungsmerkmal 72 zugeordnet ist von dem Patientenrekrutierungsmodul 48 der Datenbankumgebung 10 zu empfangen. Das Rückidentifikationsmodul 46 führt bei einem Empfang eines anonymisierten Patientendatensatzes 32 eine Rückidentifizierung des anonymisierten Patientendatensatzes 32 durch. Das Ziel der Rückidentifizierung ist eine Zuordnung eines anonymisierten Patientendatensatzes 32 zu einem Patientendatensatz 14. Die Datenbankumgebung 10 weist ein Anzeigemodul 54 auf. Das Anzeigemodul 54 ist zu einer visuellen Anzeige zumindest von mittels des Rückidentifikationsmoduls 46 rückidentifizierten Patientendatensätzen 14 vorgesehen.

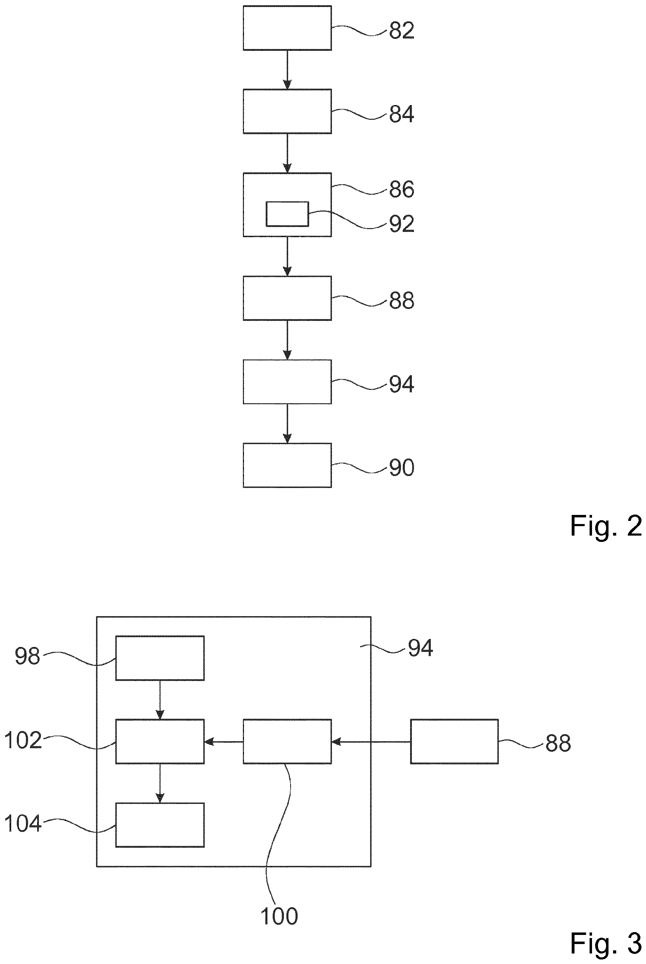

Bei dem in Fig. 2 gezeigten Verfahren zur Rekrutierung von Patienten werden in zumindest einem Verfahrensschritt 82 Patientendatensätze 14 mittels des Anonymisierungsmoduls 30 anonymisiert. In zumindest einem weiteren Verfahrensschritt 84 werden die anonymisierten Patientendatensätze 32 in der anonymisierten Patientendatenbank 60 abgespeichert. In zumindest einem weiteren Verfahrensschritt 86 werden die anonymisierten Patientendatensätze 32 mittels der Datentransporteinheit 24 versendet. In zumindest einem weiteren Verfahrensschritt 92 wird der Datentransfer von dem Beschränkungsmodul 26 auf von der Datenbankumgebung 10 ausgehende Outbound Verbindungen 28 beschränkt. In zumindest einem weiteren Verfahrensschritt 88 werden die anonymisierten Patientendatensätze 32 von dem Datenempfangsmodul 76 des Patientenrekrutierungsservers 20 empfangen. In zumindest einem weiteren Verfahrensschritt 94 wird eine Patientenrekrutierung von dem Patientenrekrutierungsmodul 22 mittels eines Durchsuchens der anonymisierten Patientendatensätze 32 in Bezug auf zumindest ein Rekrutierungsmerkmal 72 durchgeführt. In zumindest einem weiteren Verfahrensschritt 90 wird eine Rückidentifikation mittels der Rückidentifikationseinheit 78 von bei der Patientenrekrutierung aufgefundenen passenden Kandidaten durchgeführt.

Fig. 3 zeigt ein Ablaufdiagramm zumindest teilweise innerhalb des Verfahrensschritts 94 ablaufender Verfahrensschritte. In zumindest einem Verfahrensschritt 98 empfängt das Patientenrekrutierungsmodul 22 Rekrutierungsmerkmale 72 mittels der Internetschnittstelle 44 und speichert sie in der Rekrutierungsmerkmalsdatenbank 70 ab. In zumindest einem weiteren Verfahrensschritt 100 werden mittels des Datensatzabgleichmoduls 40 bereits in der Patientenrekrutierungsdatenbank 42 abgelegte anonymisierte Patientendatensätze 32 durch neu empfangene aktualisierte anonymisierte Patientendatensätze 32 ersetzt. In zumindest einem weiteren Verfahrensschritt 102 werden die anonymisierten Patientendatensätze 32 der Patientenrekrutierungsdatenbank 42 von dem Patientenrekrutierungsmodul 22 auf Übereinstimmungen mit zumindest einem Rekrutierungsmerkmal 72 durchsucht. In zumindest einem weiteren Verfahrensschritt 104 werden anonymisierte Patientendatensätze 32, welche zumindest einem Rekrutierungsmerkmal 72 zugeordnet werden konnten in der Kandidatenliste 80 abgespeichert.

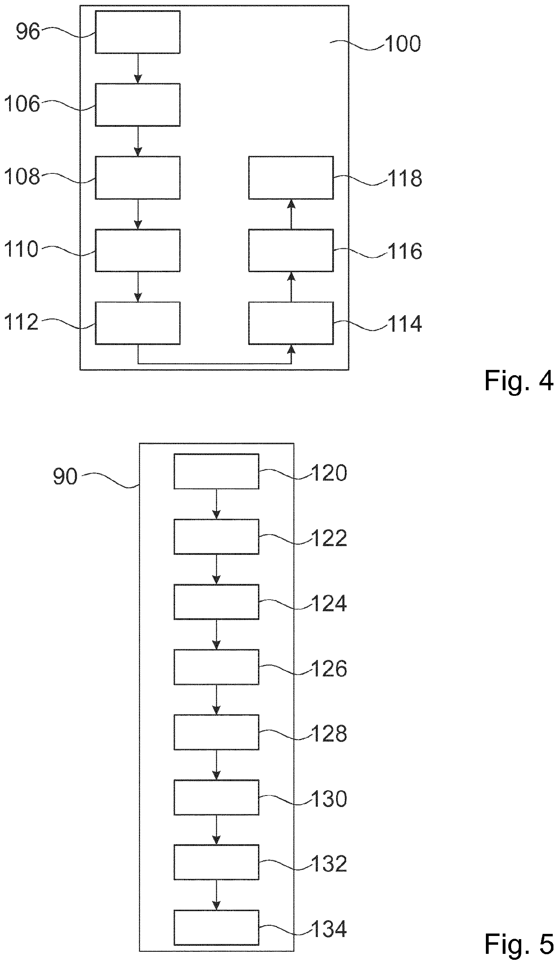

Fig. 4 zeigt ein Ablaufdiagramm zumindest teilweise innerhalb des Verfahrensschritts 100 ablaufender Verfahrensschritte. In zumindest einem Verfahrensschritt 96 wird ein Patientendatensatz 14 in der Patientendatenbank 18 aktualisiert, beispielsweise durch eine neu zu einem Behandlungsverlauf 16 hinzugefügte Diagnose. In zumindest einem weiteren Verfahrensschritt 106 wird der aktualisierte Patientendatensatz 14 in der Patientendatenbank 18 abgespeichert und dadurch der Patientendatensatz 14 überschrieben. In zumindest einem weiteren Verfahrensschritt 108 wird der aktualisierte Patientendatensatz 14 mittels des Anonymisierungsmoduls 30 anonymisiert. In zumindest einem weiteren Verfahrensschritt 110 wird der aktualisierte anonymisierte Patientendatensatz 32 zusammen mit dem vorherigen zu demselben Patienten zugehörigen anonymisierten Patientendatensatz 32 in der anonymisierten Patientendatenbank 60 abgespeichert. In zumindest einem weiteren Verfahrensschritt 112 wird der aktualisierte anonymisierte Patientendatensatz 32 zusammen mit dem vorherigen zu demselben Patienten zugehörigen anonymisierten Patientendatensatz 32 mittels der Datentransporteinheit 24 an den Patientenrekrutierungsserver 20 gesendet. In zumindest einem weiteren Verfahrensschritt 114 empfängt der Patientenrekrutierungsserver 20 den aktualisierten anonymisierten Patientendatensatz 32 zusammen mit dem vorherigen zu demselben Patienten zugehörigen anonymisierten Patientendatensatz 32 mittels dem Datenempfangsmodul 76. In zumindest einem weiteren Verfahrensschritt 116 gleicht das Datensatzabgleichmodul 40 die empfangenen anonymisierten Patientendatensätze 32 mit den in der Patientenrekrutierungsdatenbank 42 gespeicherten anonymisieren Patientendatensätzen 32 ab. In zumindest einem weiteren Verfahrensschritt 118 überschreibt das Datensatzabgleichmodul 40 einen übereinstimmenden anonymisierten Patientendatensatz 32 mit dem aktualisierten anonymisierten Patientendatensatz 32.

Fig. 4 zeigt ein Ablaufdiagramm zumindest teilweise innerhalb des Verfahrensschritts 90 ablaufender Verfahrensschritte. In zumindest einem Verfahrensschritt 120 wird von dem Patientenrekrutierungsmodul 48 der Datenbankumgebung 10 mittels der Outbound Verbindung 28 zumindest ein anonymisierter Patientendatensatz 32, welchem mittels dem Patientenrekrutierungsmodul 22 des Patientenrekrutierungsservers 20 zumindest ein Rekrutierungsmerkmal 72, zumindest in dem Verfahrensschritt 94 zugeordnet wurde von dem Patientenrekrutierungsserver 20 angefordert. In zumindest einem weiteren Verfahrensschritt 122 wird von dem Patientenrekrutierungsmodul 48 der Datenbankumgebung 10 ein angeforderter anonymisierter Patientendatensatz 32, welchem ein Rekrutierungsmerkmal 72 zugeordnet wurde empfangen. In zumindest einem weiteren Verfahrensschritt 124 wird von dem Identifikatormodul 34 ein anonymisierter Identifikator auf Basis des empfangenen anonymisierten Patientendatensatzes 32 berechnet. In zumindest einem weiteren Verfahrensschritt 126 werden die in der Tabelle 62 abgespeicherten anonymisierten Identifikatoren entschlüsselt. In zumindest einem weiteren Verfahrensschritt 128 werden die, insbesondere entschlüsselten, in der Tabelle 62 abgespeicherten anonymisierten Identifikatoren mit dem in Verfahrensschritt 124 berechnetem Identifikator abgeglichen. In zumindest einem weiteren Verfahrensschritt 130 werden mittels übereinstimmenden anonymisierten Identifikatoren zugehörige Patientendatensätze 14 aus der Tabelle 62 bestimmt. In zumindest einem weiteren Verfahrensschritt 132 werden die in Verfahrensschritt 130 bestimmten Patientendatensätze 14 für eine visuelle Anzeige an das Anzeigemodul 54 übertragen. In zumindest einem weiteren Verfahrensschritt 134 zeigt das Anzeigemodul 54 die zu den Rekrutierungsmerkmalen 72 einer klinischen Studie passenden unanonymisierten Patientendatensätze 14 an.

### Bezugszeichen

- 10: Datenbankumgebung
- 12: Datenbankmodul
- 14: Patientendatensatz
- 16: Behandlungsverlauf
- 18: Patientendatenbank
- 20: Patientenrekrutierungsserver
- 22: Patientenrekrutierungsmodul
- 24: Datentransporteinheit
- 26: Beschränkungsmodul
- 28: Outbound Verbindung
- 30: Anonymisierungsmodul
- 32: Anonymisierter Patientendatensatz
- 34: Identifikatormodul
- 36: Zuordnungsmodul
- 38: Verschlüsselungsmodul
- 40: Datensatzabgleichmodul
- 42: Patientenrekrutierungsdatenbank
- 44: Internetschnittstelle
- 46: Rückidentifikationsmodul
- 48: Patientenrekrutierungsmodul
- 50: Patientenrekrutierungsserverseite
- 52: Datenbankumgebungsseite
- 54: Anzeigemodul
- 56: Sender
- 58: Empfänger
- 60: Anonymisierte Patientendatenbank
- 62: Tabelle
- 64: Datenbankserver
- 66: Beschränkungsmodul
- 68: Cloud
- 70: Rekrutierungsmerkmalsdatenbank
- 72: Rekrutierungsmerkmal
- 74: Outbound Verbindung
- 76: Datenempfangsmodul
- 78: Rückidentifikationseinheit
- 80: Kandidatenliste
- 82: Verfahrensschritt
- 84: Verfahrensschritt
- 86: Verfahrensschritt
- 88: Verfahrensschritt
- 90: Verfahrensschritt
- 92: Verfahrensschritt
- 94: Verfahrensschritt
- 96: Verfahrensschritt
- 98: Verfahrensschritt
- 100: Verfahrensschritt
- 102: Verfahrensschritt
- 104: Verfahrensschritt
- 106: Verfahrensschritt
- 108: Verfahrensschritt
- 110: Verfahrensschritt
- 112: Verfahrensschritt
- 114: Verfahrensschritt
- 116: Verfahrensschritt
- 118: Verfahrensschritt
- 120: Verfahrensschritt
- 122: Verfahrensschritt
- 124: Verfahrensschritt
- 126: Verfahrensschritt
- 128: Verfahrensschritt
- 130: Verfahrensschritt
- 132: Verfahrensschritt
- 134: Verfahrensschritt

## Patentansprüche

1. Patientenrekrutierungssystem, insbesondere für klinische Studien,
- mit zumindest einer, in einem speziell gesicherten und zugangsbeschränkten Bereich eines Intranets eines Spitalinformationssystems angeordneten Datenbankumgebung (10),
welche zumindest ein Datenbankmodul (12) aufweist, das zumindest dazu vorgesehen ist, zumindest Patientendatensätze (14) mit zumindest einem Behandlungsverlauf (16) zumindest eines Patienten in einer Patientendatenbank (18) abzuspeichern,
welche ein Anonymisierungsmodul (30) aufweist, welches dazu vorgesehen ist, Patientendatensätze (14) in anonymisierte Patientendatensätze (32) umzuwandeln, so dass die durch das Anonymisierungsmodul (30) anonymisierten Patientendatensätze (32) frei sind von eindeutig einem Patienten zuordenbaren Identifikatoren und/oder Erkennungsmerkmalen,
welche ein Identifikatormodul (34) aufweist, welches auf Basis eines anonymisierten Patientendatensatzes (32) einen eindeutigen anonymisierten Identifikator berechnet, wobei der eindeutige anonymisierte Identifikator als eine eindeutige Prüfsumme ausgebildet ist, welche zeitlich veränderlich ausgebildet ist, so dass sich bei einer Veränderung eines anonymisierten Patientendatensatzes (32) auch der anonymisierte Identifikator verändert,
und welche ein Zuordnungsmodul (36) aufweist, welches den anonymisierten Identifikator einem dem anonymisierten Patientendatensatz (32) zugehörigen Patientendatensatz (14) aus der Patientendatenbank (18) zuordnet,
- mit zumindest einem logistisch von der Datenbankumgebung (10) getrennt angeordneten Patientenrekrutierungsserver (20),
welcher zumindest ein Patientenrekrutierungsmodul (22) aufweist, welches dazu vorgesehen ist, anonymisierte Patientendatensätze mit Behandlungsverläufen(16) zu durchsuchen und zumindest mit zumindest einem Rekrutierungsmerkmal (72) abzugleichen, und
- mit einer Datentransporteinheit (24) zu einem Datentransfer zumindest der anonymisierten Patientendatensätze zwischen der Datenbankumgebung (10) und dem Patientenrekrutierungsserver (20),
wobei die Datentransporteinheit (24) ein Beschränkungsmodul (26) aufweist, welches den Datentransfer auf von der Datenbankumgebung (10) ausgehende Outbound Verbindungen (28) beschränkt, so dass der Datentransfer zwischen der Datenbankumgebung (10) und dem Patientenrekrutierungsserver (20) ausschließlich von einer Datenbankumgebungsseite (52) aus steuerbar ist, wobei das Beschränkungsmodul (26) einen Versand der als eindeutige Prüfsummen ausgebildeten eindeutigen anonymisierten Identifikatoren unterbindet, und die von dem Identifikatormodul (34) der Datenbankumgebung (10) erzeugten und als eindeutige Prüfsummen ausgebildeten eindeutigen anonymisierten Identifikatoren dauerhaft in der Datenbankumgebung (10) verbleiben,
wobei das Identifikatormodul (34) bei einer Aktualisierung, eines bereits in einer anonymisierten Patientendatenbank (60) des Datenbankmoduls (12) vorhandenen, bereits mit einem anonymisierten Identifikator versehenen anonymisierten Patientendatensatzes (32) dem zugehörigen aktualisierten anonymisierten Patientendatensatz einen eindeutigen aktualisierten, auf Basis des zugehörigen aktualisierten anonymisierten Patientendatensatzes berechneten anonymisierten Identifikator, welcher verschieden ist von dem anonymisierten Identifikator, des bereits vor der Aktualisierung in der anonymisierten Patientendatenbank (60) des Datenbankmoduls (12) vorhandenen anonymisierten Patientendatensatzes (32) zuordnet,
wobei die Datentransporteinheit (24) bei einer Übertragung eines aktualisierten anonymisierten Patientendatensatzes von der Datenbankumgebung (10) zu dem Patientenrekrutierungsserver (20) einen ursprünglichen anonymisierten Patientendatensatz mitüberträgt,
wobei der Patientenrekrutierungsserver (20) ein Datensatzabgleichmodul (40) aufweist, welches bei einem Empfang eines bereits vorhandenen anonymisierten Patientendatensatzes eine Übereinstimmung der anonymisierten Patientendatensätze erkennt und ein Überschreiben des vorhandenen anonymisierten Patientendatensatzes mit dem zusammen empfangenen aktualisierten anonymisierten Patientendatensatz in einer Patientenrekrutierungsdatenbank (42) des Patientenrekrutierungsservers (20) veranlasst,
wobei der Patientenrekrutierungsserver (20) ein weiteres Identifikatormodul aufweist, welches dazu vorgesehen ist, von der Datentransporteinheit (24) empfangenen anonymisierten Patientendatensätzen anonymisierte Identifikatoren zuzuordnen, welche zu dem Auffinden der Übereinstimmung von identischen anonymisierten Patientendatensätzen verwendet werden, wobei ein Datenbankeintrag der Patientenrekrutierungsdatenbank (42) zumindest einen aktualisierten, anonymisierten Patientendatensatz und zumindest einen von dem Identifikatormodul (34) des Patientenrekrutierungsservers (20) berechneten anonymisierten Identifikator umfasst,
wobei zu der Berechnung der jeweiligen anonymisierten Identifikatoren zumindest eine Uhrzeit zumindest eine, insbesondere der Uhrzeit zugeordnete, medizinische Diagnose, zumindest eine, insbesondere der Uhrzeit zugeordnete, Dosierung, zumindest einen, insbesondere der Uhrzeit zugeordneten, Laborwert, und/oder zumindest eine, insbesondere der Uhrzeit zugeordnete, Medikation herangezogen wird.

2. Patientenrekrutierungssystem nach Anspruch 1, **gekennzeichnet durch** ein Verschlüsselungsmodul (38) der Datenbankumgebung (10), welches zumindest die, zumindest einem anonymisierten Patientendatensatz (32) zugehörigen, anonymisierten Identifikatoren verschlüsselt.

3. Patientenrekrutierungssystem nach Anspruch 1 oder 2, **dadurch gekennzeichnet, dass** der Patientenrekrutierungsserver (20) zumindest eine Internetschnittstelle (44) zumindest zu einem Empfang von zumindest einem Rekrutierungsmerkmal (72) aufweist.

4. Patientenrekrutierungssystem nach Anspruch 1, **dadurch gekennzeichnet, dass** ein Patientenrekrutierungsmodul (48) der Datenbankumgebung (10) mittels der Outbound Verbindung (28) der Datentransporteinheit (24) zumindest einen anonymisierten Patientendatensatz (32), welchem mittels des Patientenrekrutierungsmoduls (22) des Patientenrekrutierungsservers (20) zumindest ein Rekrutierungsmerkmal (72), zugeordnet ist von dem Patientenrekrutierungsserver (20) anfordert.

5. Patientenrekrutierungssystem nach Anspruch 4, **dadurch gekennzeichnet, dass** ein Rückidentifikationsmodul (46) der Datenbankumgebung (10) bei einem Empfang eines durch das Patientenrekrutierungsmodul (48) der Datenbankumgebung (10) angeforderten anonymisierten Patientendatensatzes (32) eine Rückidentifizierung des anonymisierten Patientendatensatzes (32) durchführt.

6. Verfahren zur Rekrutierung von Patienten, insbesondere für klinische Studien, mittels eines Patientenrekrutierungssystems, insbesondere nach einem der vorhergehenden Ansprüche,
- mit zumindest einer in einem speziell gesicherten und zugangsbeschränkten Bereich eines Intranets eines Spitalinformationssystems angeordneten Datenbankumgebung (10),
die zumindest ein Datenbankmodul (12) aufweist, welches dazu vorgesehen ist, zumindest Patientendatensätze (14) mit zumindest einem Behandlungsverlauf (16) zumindest eines Patienten in einer Patientendatenbank (18) abzuspeichern,
welche ein Anonymisierungsmodul (30) aufweist, welches dazu vorgesehen ist, Patientendatensätze (14) in anonymisierte Patientendatensätze (32) umzuwandeln, so dass die durch das Anonymisierungsmodul (30) anonymisierten Patientendatensätze (32) frei sind von eindeutig einem Patienten zuordenbaren Identifikatoren und/oder Erkennungsmerkmalen,
welche ein Identifikatormodul (34) aufweist, welches auf Basis eines anonymisierten Patientendatensatzes (32) einen eindeutigen anonymisierten Identifikator berechnet, wobei der eindeutige anonymisierte Identifikator als eine eindeutige Prüfsumme ausgebildet ist, welche zeitlich veränderlich ausgebildet ist, so dass sich bei einer Veränderung eines anonymisierten Patientendatensatzes (32) auch der anonymisierte Identifikator sich verändert, und
welche ein Zuordnungsmodul (36) aufweist, welches den anonymisierten Identifikator einem dem anonymisierten Patientendatensatz (32) zugehörigen Patientendatensatz (14) aus der Patientendatenbank (18) zuordnet,
- mit zumindest einem logistisch von der Datenbankumgebung (10) getrennt angeordneten Patientenrekrutierungsserver (20),
welcher zumindest ein Patientenrekrutierungsmodul (22) aufweist, welches dazu vorgesehen ist, anonymisierte Patientendatensätze mit Behandlungsverläufen (16) zu durchsuchen und zumindest mit zumindest einem Rekrutierungsmerkmal (72) abzugleichen, und
- mit einer Datentransporteinheit (24) zu einem Datentransfer der anonymisierten Patientendatensätze zwischen der Datenbankumgebung (10) und dem Patientenrekrutierungsserver (20),
wobei der Datentransfer von einem Beschränkungsmodul (26) der Datentransporteinheit (24) auf von der Datenbankumgebung (10) ausgehende Outbound Verbindungen (28) derart beschränkt wird, dass der Datentransfer zwischen der Datenbankumgebung (10) und dem Patientenrekrutierungsserver (20) ausschließlich von einer Datenbankumgebungsseite (52) aus steuerbar ist, wobei von dem Beschränkungsmodul (26) ein Versand der als eindeutige Prüfsummen ausgebildeten eindeutigen anonymisierten Identifikatoren unterbunden wird, und die von dem Identifikatormodul (34) der Datenbankumgebung (10) erzeugten und als eindeutige Prüfsummen ausgebildeten eindeutigen anonymisierten Identifikatoren dauerhaft in der Datenbankumgebung (10) verbleiben,
wobei das Identifikatormodul (34) bei einer Aktualisierung, eines bereits in einer anonymisierten Patientendatenbank (60) des Datenbankmoduls (12) vorhandenen, bereits mit einem anonymisierten Identifikator versehenen anonymisierten Patientendatensatzes (32) dem zugehörigen aktualisierten anonymisierten Patientendatensatz einen eindeutigen aktualisierten, auf Basis des zugehörigen aktualisierten anonymisierten Patientendatensatzes berechneten anonymisierten Identifikator, welcher verschieden ist von dem anonymisierten Identifikator des bereits vor der Aktualisierung in der anonymisierten Patientendatenbank (60) des Datenbankmoduls (12) vorhandenen anonymisierten Patientendatensatzes (32), zuordnet,
wobei die Datentransporteinheit (24) bei einer Übertragung eines aktualisierten anonymisierten Patientendatensatzes von der Datenbankumgebung (10) zu dem Patientenrekrutierungsserver (20) einen ursprünglichen anonymisierten Patientendatensatz mitüberträgt,
wobei der Patientenrekrutierungsserver (20) ein Datensatzabgleichmodul (40) aufweist, welches bei einem Empfang eines bereits vorhandenen anonymisierten Patientendatensatzes eine Übereinstimmung der anonymisierten Patientendatensätze erkennt und ein Überschreiben des vorhandenen anonymisierten Patientendatensatzes mit dem zusammen empfangenen aktualisierten anonymisierten Patientendatensatz in einer Patientenrekrutierungsdatenbank (42) des Patientenrekrutierungsservers (20) veranlasst,
wobei der Patientenrekrutierungsserver (20) ein weiteres Identifikatormodul aufweist, welches von der Datentransporteinheit (24) empfangenen anonymisierten Patientendatensätzen anonymisierte Identifikatoren zuordnet, welche zu dem Auffinden der Übereinstimmung von identischen anonymisierten Patientendatensätzen verwendet werden,
wobei ein Datenbankeintrag der Patientenrekrutierungsdatenbank (42) zumindest einen aktualisierten, anonymisierten Patientendatensatz und zumindest einen von dem Identifikatormodul (34) des Patientenrekrutierungsservers (20) berechneten anonymisierten Identifikator umfasst,
wobei zu der Berechnung der jeweiligen anonymisierten Identifikatoren zumindest eine Uhrzeit
und zumindest eine, insbesondere der Uhrzeit zugeordnete, medizinische Diagnose, zumindest eine, insbesondere der Uhrzeit zugeordnete, Dosierung, zumindest einen, insbesondere der Uhrzeit zugeordneten, Laborwert, und/oder zumindest eine, insbesondere der Uhrzeit zugeordnete, Medikation herangezogen wird.

## Claims

1. Patient recruitment system, in particular for clinical studies,
- with at least one database environment (10) arranged in a specially secured access-restricted region of an intranet of a hospital information system,
comprising at least one database module (12) that is at least configured to store in a patient database (18) at least patient data records (14) containing at least one course of treatment (16) of at least one patient,
comprising an anonymization module (30), which is configured to convert patient data records (14) into anonymized patient data records (32), such that the patient data records (32) anonymized by the anonymization module (30) are free of identifiers and/or identifying features that are unambiguously attributable to a patient,
comprising an identifier module (34) that calculates, on the basis of an anonymized patient data record (32), an unambiguous anonymized identifier, the unambiguous anonymized identifier being implemented as an unambiguous checksum that is implemented to be temporally modifiable, such that the anonymized identifier changes when an anonymized patient data record (32) changes,
and comprising an assignment module (36), which assigns the anonymized identifier to a patient data record (14) from the patient database (18) which is associated with the anonymized patient data record (32),
- with at least one patient recruitment server (20) arranged in logistic separation from the database environment (10),
which comprises at least one patient recruitment module (22) that is configured to search anonymized patient data records (32) containing courses of treatment (16) and to compare them with at least one recruitment feature (72), and
- with a data transport unit (24) for a data transfer at least of the anonymized patient data records (32) between the database environment (10) and the patient recruitment server (20),
wherein the data transport unit (24) has a restriction module (26) restricting the data transfer to outbound connections (28) originating from the database environment (10), such that the data transfer between the database environment (10) and the patient recruitment server (20) can be controlled solely from a database-environment end (52),
wherein the restriction module (26) prevents an emission of the unambiguous anonymized identifiers implemented as unambiguous checksums, and the unambiguous anonymized identifiers generated by the identifier module (34) of the database environment (10) and implemented as unambiguous checksums remain in the database environment (10) permanently,
wherein, when an anonymized patient data record (32) is updated that already exists in an anonymized patient database (60) of the database module (12) and is already provided with an anonymized identifier, the identifier module (34) assigns to the associated updated anonymized patient data record an unambiguous updated anonymized identifier calculated on the basis of the associated updated anonymized patient data record, which is different from the anonymized identifier of the anonymized patient data record (32) already existing in the anonymized patient database (60) of the database module (12),
wherein in a transmission of an updated anonymized patient data record from the database environment (10) to the patient recruitment server (20), the data transport unit (24) jointly transmits an original anonymized patient data record, wherein the patient recruitment server (20) comprises a data-record comparison module (40), which on receipt of an already existing anonymized patient data record detects a congruency of the anonymized patient data records and causes the existing anonymized patient data record to be overwritten with the jointly received updated anonymized patient data record in a patient recruitment database (42) of the patient recruitment server (20),
wherein the patient recruitment server (20) comprises a further identifier module, which is configured to assign anonymized identifiers, which are used to find the congruency of identical anonymized patient data records, to anonymized patient data records received from the data transport unit (24),
wherein a database entry of the patient recruitment database (42) comprises at least one updated anonymized patient data record and at least one anonymized identifier calculated by the identifier module (34) of the patient recruitment server (20),
wherein
at least a time of day and
at least one medical diagnosis, which is in particular associated with the time of day,
at least one dosage, which is in particular associated with the time of day,
at least one laboratory value, which is in particular associated with the time of day, and/or
at least one medication, which is in particular associated with the time of day, are used for the calculation of the respective anonymized identifiers.

2. Patient recruitment system according to claim 1, **characterized by** an encryption module (38) of the database environment (10), which encrypts at least the anonymized identifiers associated with at least one anonymized patient data record (32).

3. Patient recruitment system according to claim 1 or 2, **characterized in that** the patient recruitment server (20) comprises at least one Internet port (44) at least for receiving at least one recruitment feature (72).

4. Patient recruitment system according to claim 1, **characterized in that** a patient recruitment module (48) of the database environment (10) requests from the patient recruitment server (20), by means of the outbound connection (28) of the data transport unit (24), at least one anonymized patient data record (32), which has been assigned at least one recruitment feature (72) by the patient recruitment module (22) of the patient recruitment server (20).

5. Patient recruitment system according to claim 4, **characterized in that** on receipt of an anonymized patient data record (32) requested by the patient recruitment module (48) of the database environment (10), a reverse identification module (46) of the database environment (10) performs a reverse identification of the anonymized patient data record (32).

6. Method for recruiting patients, in particular for clinical studies, by means of a patient recruitment system, in particular according to one of the preceding claims,
- with at least one database environment (10) that is arranged in a specially secured and access-restricted region of an intranet of a hospital information system,
comprising at least one database module (12) that is configured to store in a patient database (18) at least patient data records (14) containing at least one course of treatment (16) of at least one patient,
comprising an anonymization module (30), which is configured to convert patient data records (14) into anonymized patient data records (32), such that the patient data records (32) anonymized by the anonymization module (30) are free of identifiers and/or identifying features which can be unambiguously attributed to a patient,
comprising an identifier module (34) that calculates, on the basis of an anonymized patient data record (32), an unambiguous anonymized identifier, the unambiguous anonymized identifier being implemented as an unambiguous checksum, which is implemented to be temporally modifiable, such that the anonymized identifier changes when an anonymized patient data record (32) changes,
and comprising an assignment module (36), which assigns the anonymized identifier to a patient data record (14) from the patient database (18) which is associated with the anonymized patient data record (32),
- with at least one patient recruitment server (20) arranged in logistic separation from the database environment (10),
comprising at least one patient recruitment module (22) that is configured to search anonymized patient data records containing courses of treatment (16) and to compare them with at least one recruitment feature (72), and
- with a data transport unit (24) for a data transfer of the anonymized patient data records (32) between the database environment (10) and the patient recruitment server (20),
wherein the data transfer is restricted to outbound connections (28) originating from the database environment (10) by a restriction module (26) of the data transport unit (24) in such a way that the data transfer between the database environment (10) and the patient recruitment server (20) can be controlled solely from a database-environment end (52),
wherein an emission of the unambiguous anonymized identifiers implemented as unambiguous checksums is prevented by the restriction module (26), and
the unambiguous anonymized identifiers generated by the identifier module (34) of the database environment (10) and implemented as unambiguous checksums remain in the database environment (10) permanently,
wherein, when an anonymized patient data record (32) is updated that already exists in an anonymized patient database (60) of the database module (12) and is already provided with an anonymized identifier, the identifier module (34) assigns to the associated updated anonymized patient data record an unambiguous updated anonymized identifier calculated on the basis of the associated updated anonymized patient data record, which is different from the anonymized identifier of the anonymized patient data record (32) already existing in the anonymized patient database (60) of the database module (12) before the update,
wherein in a transmission of an updated anonymized patient data record from the database environment (10) to the patient recruitment server (20), the data transport unit (24) jointly transmits an original anonymized patient data record, wherein the patient recruitment server (20) comprises a data-record comparison module (40), which on receipt of an already existing anonymized patient data record detects a congruency of the anonymized patient data records and causes the existing anonymized patient data record to be overwritten with the jointly received updated anonymized patient data record in a patient recruitment database (42) of the patient recruitment server (20),
wherein the patient recruitment server (20) comprises a further identifier module, which assigns anonymized identifiers, which are used to find the congruency of identical anonymized patient data records, to anonymized patient data records received from the data transport unit (24),
wherein a database entry of the patient recruitment database (42) comprises at least one updated anonymized patient data record and at least one anonymized identifier calculated by the identifier module (34) of the patient recruitment server (20),
wherein at least a time of day
and at least one medical diagnosis, which is in particular associated with the time of day,
at least one dosage, which is in particular associated with the time of day,
at least one laboratory value, which is in particular associated with the time of day,
and/or at least one medication, which is in particular associated with the time of day, are used for the calculation of the respective anonymized identifiers.

## Revendications

1. Système de recrutement des patients, notamment pour des essais cliniques,
- avec au moins un environnement de base de données (10) disposé dans une zone accès-restreint spécifiquement sécurisée d'un intranet d'un système d'information-hôpital,
ledit environnement de base de données (10) comprenant au moins un module de base de données (12) qui est au moins configuré pour un stockage dans une base de données-patient (18) au moins des jeux de données-patient (14) avec au moins un cours de traitement (16) d'au moins un patient,
comprenant un module d'anonymisation (30) qui est configuré à convertir des jeux de données-patient (14) dans des jeux de données-patient anonymisés (32), en sorte que les jeux de données-patient anonymisés (32) anonymisés par le module d'anonymisation (30) soient libres des identifiants et/ou caractéristiques distinctives qui sont attribuables à un patient sans ambiguïté,
comprenant un module d'identifiant (34) qui calcule un identifiant anonymisé univoque sur la base d'un jeu de données-patient anonymisé (32), l'identifiant anonymisé univoque étant réalisé comme somme de contrôle univoque formée d'une manière temporellement variable, en sorte qu'avec un changement d'un jeu de données-patient anonymisé (32), l'identifiant anonymisé change aussi,
et comprenant un module d'attribution (36) qui attribue l'identifiant anonymisé à un jeu de données-patient (14) de la base de données-patient (18) appartenant au jeu de données-patient anonymisé (32),
- avec au moins un serveur informatique de recrutement des patients (20) localisé en séparation logistique de l'environnement de base de données (10), ledit serveur informatique de recrutement des patients (20) comprenant au moins un module de recrutement des patients (22) qui est configuré à chercher des jeux de données-patient anonymisés avec des cours de traitement (16), au moins faisant un recoupement desdits jeux de données-patient anonymisés avec au moins une caractéristique de recrutement (72), et
- avec une unité de transport de données (24) pour un transfert de données au moins des jeux de données-patient anonymisés entre l'environnement de base de données (10) et le serveur informatique de recrutement des patients (20),
ladite unité de transport de données (24) comprenant un module de limitation (26) qui limite le transfert de données à des liaisons sortantes (28) de l'environnement de base de données (10), en sorte que le transfert de données entre l'environnement de base de données (10) et le serveur informatique de recrutement des patients (20) puisse être commandé exclusivement d'un côté environnement de base de données (52),
le module de limitation (26) empêchant l'envoi des identifiants anonymisés univoques qui sont réalisés comme sommes de contrôle univoques, et
les identifiants anonymisés univoques, générés par le module d'identifiant (34) de l'environnement de base de données (10) et réalisés comme sommes de contrôle univoques, restant dans l'environnement de base de données (10) perpétuellement,
où - en cas d'une actualisation d'un jeu de données-patient anonymisé (32) qui existe déjà dans une base de données-patient anonymisée (60) du module de base de données (12) et est déjà pourvu d'un identifiant anonymisé - le module d'identifiant (34) attribue au jeu de données-patient anonymisé correspondant actualisé un identifiant anonymisé univoque actualisé qui est calculé sur la base du jeu de données-patient anonymisé correspondant actualisé et qui diffère de l'identifiant anonymisé du jeu de données-patient anonymisé (32) qui était déjà présent dans la base de données-patient anonymisée (60) du module de base de données (12) avant l'actualisation,
où - dans un transfert d'un jeu de données-patient anonymisé actualisé de l'environnement de base de données (10) au serveur informatique de recrutement des patients (20) - l'unité de transport de données (24) au même temps transfert un jeu de données-patient anonymisé original,
où le serveur informatique de recrutement des patients (20) comprend un module à recoupement de jeu de données (40), qui en recevant un jeu de données-patient anonymisé déjà existant détecte une coïncidence des jeux de données-patient anonymisés et initie un écrasement du jeu de données-patient anonymisé déjà existant par le jeu de données-patient anonymisé actualisé reçu au même temps dans une base de données de recrutement des patients (42) du serveur informatique de recrutement des patients (20),
où le serveur informatique de recrutement des patients (20) comprend un autre module d'identifiant, qui est configuré à attribuer aux jeux de données-patient anonymisés reçus de l'unité de transport de données (24) des identifiants anonymisés qui sont utilisés pour trouver la coïncidence des jeux de données-patient anonymisés identiques,
où un enregistrement dans la base de données de recrutement des patients (42) comprend au moins un jeu de données-patient anonymisé actualisé et au moins un identifiant anonymisé qui est calculé par le module d'identifiant (34) du serveur informatique de recrutement des patients (20),
où pour un calcul des identifiants anonymisés respectifs au moins une heure
et au moins une diagnose médicale, en particulier attribuée à l'heure, au moins un dosage, en particulier attribué à l'heure, au moins une valeur de laboratoire, en particulier attribué à l'heure, et/ou au moins une médication, attribuée en particulier à l'heure,
sont utilisés.

2. Système de recrutement des patients selon la revendication 1, **caractérisé par** un module d'encodage (38) de l'environnement de base de données (10) qui encode au moins les identifiants anonymisés appartenant à au moins un jeu de données-patient anonymisé (32).

3. Système de recrutement des patients selon la revendication 1 ou 2, **caractérisé en ce que** le serveur informatique de recrutement des patients (20) comprend au moins un interface internet (44) au moins pour une réception d'au moins une caractéristique de recrutement (72).

4. Système de recrutement des patients selon la revendication 1,
**caractérisé en ce que** par le biais de la liaison sortante (28) de l'unité de transport de données (24), un module de recrutement des patients (48) de l'environnement de base de données (10) demande du serveur informatique de recrutement des patients (20) au moins un jeu de données-patient anonymisé (32), auquel au moins une caractéristique de recrutement (72) a été attribuée par le module de recrutement des patients (22) du serveur informatique de recrutement des patients (20).

5. Système de recrutement des patients selon la revendication 4,
**caractérisé en ce que** - en recevant un jeu de données-patient anonymisé (32) demandé par le module de recrutement des patients (48) de l'environnement de base de données (10) - un module de réidentification (46) de l'environnement de base de données (10) exécute une réidentification du jeu de données-patient anonymisé (32).

6. Procédé pour le recrutement des patients, notamment pour des essais cliniques, par le biais d'un système de recrutement des patients, en particulier selon l'une des revendications précédentes,
- avec au moins un environnement de base de données (10) disposé dans une zone accès-restreint spécifiquement sécurisée d'un intranet d'un système d'information-hôpital,
ledit environnement de base de données (10) comprenant au moins un module de base de données (12) qui est au moins configuré pour un stockage dans une base de données-patient (18) au moins des jeux de données-patient (14) avec au moins un cours de traitement (16) d'au moins un patient,
comprenant un module d'anonymisation (30) qui est configuré à convertir des jeux de données-patient (14) dans des jeux de données-patient anonymisés (32), en sorte que les jeux de données-patient anonymisés (32) anonymisés par le module d'anonymisation (30) soient libres des identifiants et/ou caractéristiques distinctives qui sont attribuables à un patient sans ambiguïté,
comprenant un module d'identifiant (34) qui calcule un identifiant anonymisé univoque sur la base d'un jeu de données-patient anonymisé (32), l'identifiant anonymisé univoque étant réalisé comme somme de contrôle univoque formée d'une manière temporellement variable, en sorte qu'avec un changement d'un jeu de données-patient anonymisé (32), l'identifiant anonymisé change aussi,
et comprenant un module d'attribution (36) qui attribue l'identifiant anonymisé à un jeu de données-patient (14) de la base de données-patient (18) appartenant au jeu de données-patient anonymisé (32),
- avec au moins un serveur informatique de recrutement des patients (20) localisé en séparation logistique de l'environnement de base de données (10),
ledit serveur informatique de recrutement des patients (20) comprenant au moins un module de recrutement des patients (22) qui est configuré à chercher des jeux de données-patient anonymisés incluant des cours de traitement (16), au moins faisant un recoupement desdits jeux de données-patient anonymisés avec au moins une caractéristique de recrutement (72), et
- avec une unité de transport de données (24) pour un transfert de données des jeux de données-patient anonymisés entre l'environnement de base de données (10) et le serveur informatique de recrutement des patients (20),
le transfert de données étant limité à des liaisons sortantes (28) de l'environnement de base de données (10) par un module de limitation de l'unité de transport de données (24) en sorte que le transfert de données entre l'environnement de base de données (10) et le serveur informatique de recrutement des patients (20) puisse être commandé exclusivement d'un côté environnement de base de données (52),
l'envoi des identifiants anonymisés univoques qui sont réalisés comme sommes de contrôle univoques étant empêché par le module de limitation (26), et
les identifiants anonymisés univoques, générés par le module d'identifiant (34) de l'environnement de base de données (10) et réalisés comme sommes de contrôle univoques, restant dans l'environnement de base de données (10) perpétuellement,
où - en cas d'une actualisation d'un jeu de données-patient anonymisé (32) qui existe déjà dans une base de données-patient anonymisée (60) du module de base de données (12) et est déjà pourvu d'un identifiant anonymisé - le module d'identifiant (34) attribue au jeu de données-patient anonymisé correspondant actualisé un identifiant anonymisé univoque actualisé qui est calculé sur la base du jeu de données-patient anonymisé correspondant actualisé et qui diffère de l'identifiant anonymisé du jeu de données-patient anonymisé (32) qui était déjà présent dans la base de données-patient anonymisée (60) du module de base de données (12) avant l'actualisation,
où - dans un transfert d'un jeu de données-patient anonymisé actualisé de l'environnement de base de données (10) au serveur informatique de recrutement des patients (20) - l'unité de transport de données (24) au même temps transfert un jeu de données-patient anonymisé original,
où le serveur informatique de recrutement des patients (20) comprend un module à recoupement de jeu de données (40), qui en recevant un jeu de données-patient anonymisé déjà existant détecte une coïncidence des jeux de données-patient anonymisés et initie un écrasement du jeu de données-patient anonymisé déjà existant par le jeu de données-patient anonymisé actualisé reçu au même temps dans une base de données de recrutement des patients (42) du serveur informatique de recrutement des patients (20),
où le serveur informatique de recrutement des patients (20) comprend un autre module d'identifiant, qui attribue aux jeux de données-patient anonymisés reçus de l'unité de transport de données (24) des identifiants anonymisés qui sont utilisés pour trouver la coïncidence des jeux de données-patient anonymisés identiques,
où un enregistrement dans la base de données de recrutement des patients (42) comprend au moins un jeu de données-patient anonymisé actualisé et au moins un identifiant anonymisé qui est calculé par le module d'identifiant (34) du serveur informatique de recrutement des patients (20),
où pour un calcul des identifiants anonymisés respectifs au moins une heure
et au moins une diagnose médicale, en particulier attribuée à l'heure, au moins un dosage, en particulier attribué à l'heure, au moins une valeur de laboratoire, en particulier attribuée à l'heure, et/ou au moins une médication, attribuée en particulier à l'heure,
sont utilisés.
